(19) Europäisches Patentamt
European Patent Office
Office européen des brevets

(11) Numéro de publication: **0 463 945 B1**

# (12) FASCICULE DE BREVET EUROPEEN

(45) Date de publication du fascicule du brevet: **27.12.95**

(21) Numéro de dépôt: **91401679.5**

(22) Date de dépôt: **21.06.91**

(51) Int. Cl.$^6$: **C07D 263/58**, A61K 31/42, C07D 277/68, A61K 31/425, C07D 265/36, C07D 265/14, C07D 279/16, C07D 263/52, C07D 413/04

## (54) Nouvelles chalcones, leur procédé de préparation et les compositions pharmaceutiques qui les contiennent

(30) Priorité: **22.06.90 FR 9007813**

(43) Date de publication de la demande:
**02.01.92 Bulletin 92/01**

(45) Mention de la délivrance du brevet:
**27.12.95 Bulletin 95/52**

(84) Etats contractants désignés:
**AT BE CH DE DK ES FR GB GR IT LI LU NL SE**

(56) Documents cités:
**EP-A- 0 223 674**
**FR-A- 2 244 506**

**EUROPEAN JOURNAL OF MEDICINAL CHEMISTRY, vol. 24, no. 1, janvier/février 1989, pages 55-60, Paris, FR; Z. MOUSSAVI et al.: "Acyl-7 dihydro-2,3 benzoxazin-1-4 ones-3 et propriétés normolipémiantes"**

(73) Titulaire: **ADIR ET COMPAGNIE**
**1 rue Carle Hébert**
**F-92415 Courbevoie Cédex (FR)**

(72) Inventeur: **Lesieur, Daniel**
**20 rue de Verdun**
**F-59147 Gondecourt (FR)**
Inventeur: **Caignard, Daniel-Henri**
**69 bis rue Brancion**
**F-75015 Paris (FR)**
Inventeur: **Lesieur, Isabelle**
**20 rue de Verdun**
**F-59147 Gondecourt (FR)**
Inventeur: **Devissaguet, Michelle**
**14 boulevard d'Inkermann**
**F-92200 Neuilly Sur Seine (FR)**
Inventeur: **Guardiola, Béatrice**
**6 rue Edouard Nortier**
**F-92200 Neuilly Sur Seine (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

| | |
|---|---|
| **EUROPEAN JOURNAL OF MEDICINAL CHEMISTRY, vol. 9, no. 5, septembre/octobre 1974, pages 497-500, Paris, FR; J.-P. BONTE et al.: "Acyl-6 benzoxazolinones (2eme mémoire). Préparations de quelques produits de transformation"** | |

## Description

La présente invention concerne de nouvelles chalcones hétérocycliques, leur procédé de préparation et les compositions pharmaceutiques qui les contiennent.

Certaines chalcones hétérocycliques ont déjà été décrites dans le brevet Fr 73.23281 et dans Eur. J. Med. Chem. 1974, 9, (5), 497-500 comme ayant des propriétés antalgiques.

Plus récemment la demande de brevet européen 0 223 674 revendique des chalcones benzoxazinoniques à propriétés hypolipidémiantes, analgésiques, antiinflammatoires, antimicrobiennes, antifongiques, cardiovasculaires et sur le système nerveux central. La demande de brevet JP 61.087560 revendique des chalcones, notamment benzoxazolinoniques douées de propriétés antiallergiques.

La demanderesse a maintenant découvert des chalcones hétérocycliques qui tout en conservant de bonnes propriétés antiinflammatoires sont à la fois douées de remarquables propriétés antioxydantes que l'art antérieur ne mentionne pas. Les propriétés antioxydantes sont étroitement liées à la structure originale des composés de l'invention car les produits de l'art antérieur précédemment cité, structuralement les plus proches des produits de l'invention, en sont dépourvus.

Plus spécifiquement, l'invention concerne des dérivés de formule générale (I) :

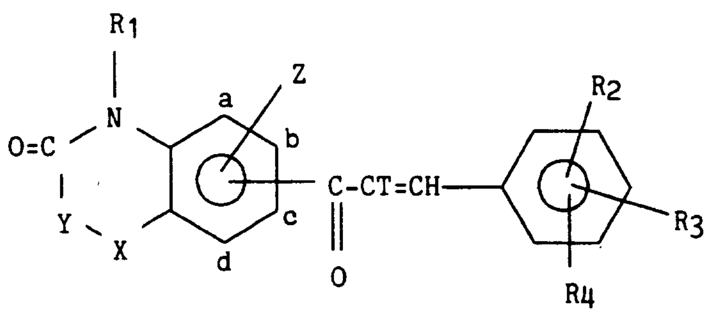

(I)

dans laquelle :

- $R_1$ représente un atome d'hydrogène ou un radical alkyle inférieur,
- X représente :
  - . un atome d'oxygène ou de soufre,
  - . un groupement $CH_2$ à la condition que dans ce cas Y représente un atome d'oxygène ou de soufre,
- Y représente :
  - . une liaison simple ou un groupement $CR_7R_8$ où $R_7$ et $R_8$ identiques ou différents représentent indépendamment l'un de l'autre un atome d'hydrogène, un groupement alkyle inférieur, un groupement phényle, un groupement phényle substitué, un groupement phényl alkyle ou un groupement phényl alkyle substitué,
  - . un atome d'oxygène ou de soufre à la condition que dans ce cas X représente un groupement $CH_2$,
- Z représente ou bien un atome d'hydrogène et dans ce cas T représente également un atome d'hydrogène ou bien Z forme avec T un chaînon $-(CH_2)_n-CH(E)-$ ou $(CH2)n-CH(CH_2E')$ avec n entier égal à 0, 1, 2 ou 3 étant entendu que dans ce cas Z est porté par un carbone voisin du carbone porteur du groupement acylé, et E ou E' représente un atome d'hydrogène, un groupement alkyle inférieur, un groupement phényle, hétéroaryle, ou phényle substitué ou hétéroaryle substitué,

le terme substitué affectant les termes phényle et hétéroaryle et phényle alkyle dans la définition de $R_7$ et $R_8$ et E et E' signifiant que ces groupements peuvent être substitués par un ou plusieurs substituants choisis parmi alkyle inférieur, alkoxy inférieur, halogène, trifluorométhyle ou hydroxyle,

- $R_2$, $R_3$ et $R_4$ identiques ou différents représentent indépendamment l'un de l'autre un groupement hydroxy ou alkoxy inférieur ou un groupement alkyle inférieur aux conditions que :
  - . lorsque X représente l'oxygène, Y représente une liaison simple et Z représente un atome d'hydrogène, l'un au moins de $R_2$, $R_3$ et $R_4$ est différent du groupement méthoxy,
  - . lorsque X représente l'oxygène, Y représente un groupement $CR_7R_8$ et Z représente un atome d'hydrogène, l'un au moins de $R_2$, $R_3$, $R_4$ n'est pas un groupement alkyle,

leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable,

étant entendu que par alkyle inférieur ou alkoxy inférieur, on entend des groupements linéaires ou ramifiés comprenant de 1 à 6 atomes de carbone,

que par hétéroaryle on entend des groupements non saturés mono ou bicycliques comprenant de 5 à 12 atomes autres que l'hydrogène et incluant dans un squelette carboné de 1 à 3 hétéroatomes choisis parmi azote, oxygène ou soufre.

Parmi les bases que l'on peut ajouter aux composés de formule (I) pour former un sel d'addition, on peut citer à titre d'exemple, les hydroxydes de sodium, potassium, calcium, ou des bases organiques comme la diéthylamine, la diéthanolamine, la triéthylamine, la benzylamine, la dicyclohexylamine, l'arginine ou des carbonates de métaux alcalins ou alcalino terreux.

Parmi les composés de l'invention, ceux pour lesquels :

$R_2$ = $R_4$ = tert.butyl, $R_3$ = OH,

ainsi que ceux pour lesquels le groupement cinnamoyle substitué est fixé en position b ou c du cycle aromatique sont préférés.

L'invention s'étend également au procédé d'obtention des composés de formule (I) caractérisé en ce que l'on fait réagir un dérivé de formule (II) :

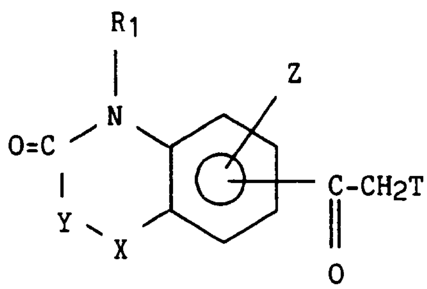

(II)

dans laquelle $R_1$, X, Y, Z et T ont la même signification que dans la formule (I),

obtenu comme décrit ci-dessous ou dans les demandes de brevets France 73.23281, 89.05655, 90.07812, Europe 0 223 674 et Pologne 85.784 (appl. 161,856, 11.04.73), et les publications Ann. Chim. (Rome) 1955, 45, 172 - Eur. J. Med. Chem. 89, 24, 5, 479-485 ; Ind. J. Chem. 1983, 22B, 1236 ; Bull. Chem. Soc. Jpn 79, 52(4), 1135-8,

en milieu organique acide avec un aldéhyde de formule (III) :

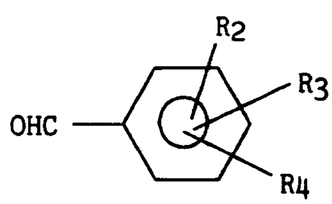

(III)

dans laquelle $R_2$, $R_3$ et $R_4$ ont la même définition que dans la formule (I),

pour conduire, après éventuelle neutralisation du milieu réactionnel, à un dérivé de formule (I), que l'on purifie si nécessaire par une technique choisie parmi chromatographie ou cristallisation, dont on sépare les isomères, le cas échéant et que l'on salifie, si on le désire, lorsque $R_1$ représente un atome d'hydrogène ou l'un au moins des groupements $R_2$, $R_3$ et $R_4$ représente un groupement hydroxyle, par une base pharmaceutiquement acceptable.

Les composés de formule (II) pour lesquels Z forme avec T un chaînon $-(CH_2)_n-CH(E)-$ ou $-(CH_2)_n-CH(CH_2E')$ appelés dérivés (II/Z), à l'exception de ceux pour lesquels X représente un atome d'oxygène, Y représente une liaison simple et E représente un groupement méthyle (E' représente un atome d'hydrogène) sont nouveaux et font partie de l'invention au même titre que les dérivés de formule (I) dont ils constituent des intermédiaires de synthèse.

Les composés de formule (II/Z) pourront être préparés par cyclisation en milieu acide de dérivés de formule (IV), lesquels ne sont pas nécessairement isolés,

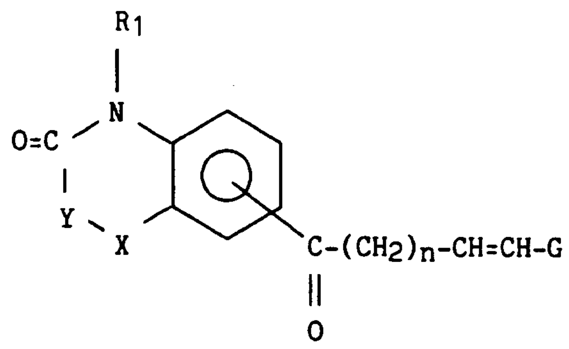

**(IV)**

avec n, Y, X et $R_1$ ayant la même définition que dans la formule (I) et G représente E ou $CH_2E'$,
dérivé de formule (IV) que l'on obtiendra,

- soit lorsque Y représente une liaison simple, par acylation en milieu acide d'un dérivé de formule (V) :

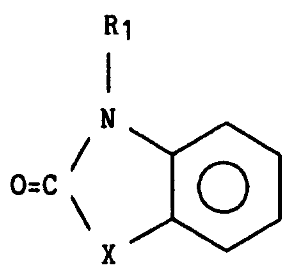

**(V)**

dans laquelle $R_1$ et X ont la même définition que dans la formule (I),
par un chlorure d'acide de formule (VI) :

$ClOC\text{-}(CH_2)_nCH=CH\text{-}E$ **(VI)**

dans laquelle n et E ont la même définition que dans la formule (I),
ou par un dérivé de formule (VII) :

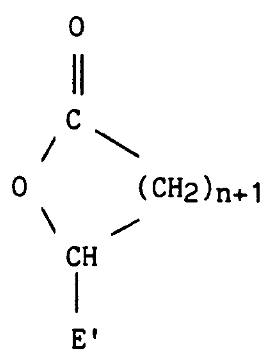

**(VII)**

dans laquelle n et E' ont la même définition que dans la formule (I),

- soit lorsque n = 0 par condensation d'un dérivé de formule (VIII) :

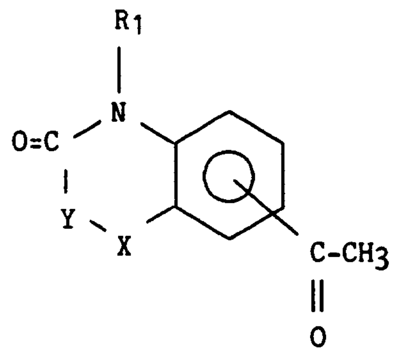

**(VIII)**

où X, Y, $R_1$ et n ont la même définition que dans la formule (I), obtenu selon les moyens classiques décrits dans la littérature, avec un aldéhyde de formule (IX) :

G-CHO **(IX)**

où G a la même définition que précédemment,

- soit, lorsque G représente un atome d'hydrogène et n = 0, par déhydrohalogénation en milieu alcalin d'un dérivé de formule (X) :

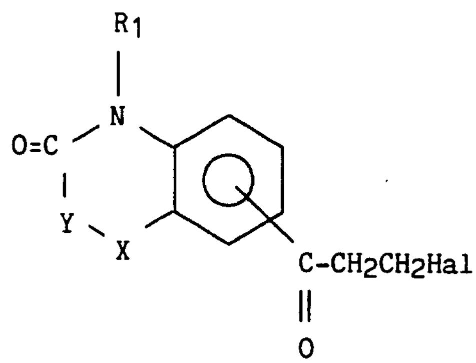

**(X)**

ou $R_1$, X, Y et n ont la même définition que précédemment et Hal représente un atome d'halogène, avec le cas échéant purification et/ou séparation des isomères.

Les composés de formule (I) possèdent des propriétés pharmacologiques intéressantes.

L'étude pharmacologique des dérivés de l'invention a en effet montré qu'ils étaient peu toxiques, doués d'activité antiinflammatoire d'un bon niveau mais surtout de remarquables propriétés antioxydantes. Ce spectre d'activité rend les composés de la présente invention intéressants dans un certain nombre d'indications telles que algies rhumatismales, névralgies, lombosciatiques, névralgies cervico-brachiales, mais surtout de la prévention des accidents ischémiques artériels périphériques et cérébro-vasculaires, l'athérosclérose, des infarctus, dans la prévention et le traitement des troubles plaquettaires, dans les hypercholestérolémies, et hypertriglycéridémies, dans le diabète non insulino dépendant ainsi que les multiples complications telles que cataractes, troubles visuels, névropathies et néphropathies. La propriété antioxydante des dérivés de l'invention permet également de les utiliser en tant que conservateur notamment d'organes.

Bien que certaines des indications des dérivés de l'invention soient parallèles à celles de certains documents de l'art antérieur (demande de brevet EP 0223674 notamment), les dérivés de l'invention se démarquent par un mode d'action totalement différent, et somme toute, complémentaire. Les dérivés de la demande EP 0223674 structuralement très proches des dérivés de l'invention se sont en effet révélés inactifs sur la quasi totalité des tests qui démontrent l'excellent pouvoir antioxydant des dérivés de l'invention.

La présente invention a également pour objet les compositions pharmaceutiques contenant les produits de formule (I) ou un de leurs sels d'addition à une base pharmaceutiquement acceptable, seuls ou en

combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique ou des traitements éventuellement associés et s'échelonne entre 0,1 centigramme et 4 grammes par 24 heures.

**PREPARATION 1 : 2,3-DIHYDRO 2,7-DIOXO 5-PHENYL CYCLOPENTA [f] BENZOXAZOLE**

Dans un ballon de 1000 ml muni d'un agitateur mécanique, placer 1000 g d'acide polyphosphorique. Dissoudre par agitation, à une température de 120°C, 119,36 g (0,45 mole) de 6-cinnamoyl benzoxazolinone décrite dans la demande Fr 73.23281 et poursuivre l'agitation pendant 55 mn en maintenant cette température. Hydrolyser le mélange réactionnel par agitation vigoureuse dans 5 volumes d'eau glacée, essorer, laver à l'eau jusqu'à neutralité des eaux de lavage et sécher. Placer le précipité obtenu, finement pulvérisé, en suspension dans du chloroforme, porter à l'ébullition pendant 2 heures. Filtrer puis évaporer le chloroforme au bain-marie sous vide. Reprendre l'opération à plusieurs reprises. Dissoudre le résidu d'évaporation dans 300 ml d'alcool absolu et évaporer à nouveau. Recristallisation dans l'alcool absolu.
Rendement : 30%
Point de fusion : 229-231°C

**PREPARATION 2 : 3-METHYL 2,3-DIHYDRO 2,7-DIOXO 5-PHENYL CYCLOPENTA [f] BENZOXAZOLE**

Procéder comme dans la préparation 1 en remplaçant la 6-cinnamoyl benzoxazolinone par la 3-méthyl 6-cinnamoyl benzoxazolinone.
Rendement : 55%
Point de fusion : 161°C

**PREPARATION 3 : 3-METHYL 6-PENTENE-2-OYL BENZOXAZOLINONE**

Agiter un mélange de 0,01 mole de 3-méthyl 6-acétyl benzoxazolinone et 0,01 mole d'aldéhyde propionique dans une solution de potasse méthanolique à 10%. Verser le milieu réactionnel dans l'eau. Essorer, laver jusqu'à neutralité, sécher et recristalliser dans un solvant convenable.

**PREPARATION 4 : 2,3-DIHYDRO 2,7-DIOXO 5-ETHYL CYCLOPENTA [f] BENZOXAZOLE**

En procédant comme dans la préparation 1, mais en remplaçant la 6-cinnamoyl benzoxazolinone par la 3-méthyl 6-pentene-2-oyl benzoxazolinone, on obtient le produit du titre.

**PREPARATION 5 : 3-METHYL 6-ACRYLOYL BENZOXAZOLINONE**

Dans une fiole à col rodé de 150 ml munie d'un réfrigérant à reflux, dissoudre 5 g (0,02 mole) de 3-méthyl 6- (3-chloropropionyl) benzoxazolinone décrite dans la demande de brevet Fr 89.02554 dans 70 ml de diméthylformamide. Introduire 2,04 g d'acétate de potassium. Chauffer sous agitation magnétique à 75°-80°C pendant 50 mn. Après refroidissement, verser le mélange réactionnel dans l'eau glacée. Agiter pendant 0,5h. Essorer le précipité obtenu, laver à l'eau, sécher et recristalliser dans le toluène.
Rendement : 80%
Point de fusion : 145°C

**PREPARATION 6 : 2,3-DIHYDRO 2,7-DIOXO 3-METHYL CYCLOPENTA [f] BENZOXAZOLE**

Dissoudre 0,03 mole de 6-acryloyl 3-méthyl benzoxazolinone dans 30 $cm^3$ d'acide sulfurique concentré (98%). Porter à la température de 40°C pendant 4 heures sous agitation magnétique. Refroidir et verser le milieu réactionnel dans 150 $cm^3$ d'eau glacée. Essorer le précipité formé, laver par de l'eau jusqu'à neutralité du filtrat, sécher et recristalliser.

**PREPARATION 7 : [4H]2,3-DIHYDRO 3,8-DIOXO 4-METHYL 6-PHENYL CYCLOPENTA [g] 1,4-BENZOXAZINE**

En procédant comme dans la préparation 1, mais en remplaçant la 6-cinnamoyl benzoxazolinone par la 7-cinnamoyl 4-méthyl [4H] 2,3 dihydro 1,4-benzoxazin-3-one décrite dans la demande de brevet EP 0223674, on obtient le produit du titre.

**PREPARATION 8 : [4H]2,3-DIHYDRO 4-METHYL 7-CINNAMOYL 1,4-BENZOTHIAZIN-3-ONE**

En procédant comme dans la préparation 3, mais en remplaçant la 3-méthyl 6-acétyl benzoxazolin-3-one par la 4-méthyl 7-acétyl [4H] 2,3-dihydro 1,4 benzothiazin-3-one et l'aldéhyde propionique par l'aldéhyde benzoïque, on obtient le produit du titre.

**PREPARATION 9 : [4H]2,3-DIHYDRO 4-METHYL 2-BENZYL 7-CINNAMOYL BENZOXAZIN-3-ONE**

A partir de la 3-méthyl 6-cinnamoyl benzoxazolinone, on prépare successivement :

. le 2-méthylamino 5-cinnamoyl phénol par hydrolyse alcaline,
. puis selon le protocole décrit dans la demande de brevet EP 0223674 et en utilisant le 2-bromo 3-phényl propionate d'éthyle, on obtient le produit du titre.

**PREPARATION 10 : [4H]2,3-DIHYDRO 3,8-DIOXO 4-METHYL 6-PHENYL CYCLOPENTA[g] [1,4]-BENZOTHIAZINE**

En procédant comme dans la préparation 1, mais en remplaçant la 6-cinnamoyl benzoxazolinone par la [4H] 2,3-dihydro 4-méthyl 7-cinnamoyl [1,4]benzothiazin-3-one obtenue dans la préparation 8, on obtient le produit titre.

**PREPARATION 11 : [4H]2,3-DIHYDRO 2-BENZYL 3,8-DIOXO 4-METHYL 6-PHEHYL CYCLOPENTA [g] [1,4]-BENZOXAZINE**

En procédant comme dans la préparation 1, mais en remplaçant la 6-cinnamoyl benzoxazolinone par la [4H]2,3-dihydro 2-benzyl 4-méthyl 7-cinnamoyl 1,4-benzoxazine 3-one décrite dans la préparation 9, on obtient le produit du titre.

Les exemples suivants illustrent l'invention et ne la limitent en aucune façon.

**EXEMPLE 1 : 5-(3',5'-DITERT.BUTYL 4'-HYDROXY CINNAMOYL) 3-METHYL BENZOXAZOLINONE**

Dans une fiole rodée de 500 ml munie d'une agitation, placer 0,02 mole de 5-acétyl 3-méthyl benzoxazolinone dans 300 ml d'éthanol saturé d'acide chlorhydrique. Ajouter lentement et sous agitation 0,02 mole de 3,5-ditert.butyl 4-hydroxy benzaldéhyde. Abandonner le milieu réactionnel sous agitation à température ambiante durant deux heures. Essorer le précipité formé, laver à l'eau jusqu'à neutralité du filtrat.

Solvant de recristallisation : toluène
Rendement : 86%
Point de fusion : 246-248 ° C

| Analyse élémentaire : | | | |
|---|---|---|---|
| | C | H | N |
| Calculée :<br>Trouvée : | 73,68<br>73,66 | 7,17<br>7,19 | 3,43<br>3,41 |

Caractéristiques spectrales :
Infrarouge :
3520 $cm^{-1}$ : $\nu$ OH
1785 $cm^{-1}$ : $\nu$ CO (O-CO-N)
Résonance magnétique nucléaire (Solvant DMSO $D_6$)

δ : 1,44 ppm, singulet, 18H (2.C($CH_3$)$_3$)
δ : 3,44 ppm, singulet, 3H, N-$CH_3$

**EXEMPLE 2 : 5-(3',5'-DITERT.BUTYL-4'-HYDROXY CINNAMOYL) BENZOXAZOLINONE**

Procéder comme dans l'exemple 1 en remplaçant la 5-acétyl-3-méthyl benzoxazolinone par la 5-acétyl benzoxazolinone. Après deux heures d'agitation, évaporer le milieu réactionnel au bain-marie sous vide, reprendre le résidu par l'eau et agiter une heure encore le précipité, laver à l'eau, sécher.

Solvant de recristallisation : toluène
Rendement : 70%
Point de fusion : 206-207 ° C

| Analyse élémentaire : | | | |
|---|---|---|---|
| | C | H | N |
| Calculée :<br>Trouvée : | 73,26<br>73,27 | 6,98<br>7,06 | 3,55<br>3,48 |

Caractéristiques spectrales :
Infrarouge :
3610 $cm^{-1}$ : ν OH
1780 $cm^{-1}$ : ν CO (O-CO-N)
1650 $cm^{-1}$ : ν CO (acyl)
1615 $cm^{-1}$ : ν C=C (éthylénique)
Résonance magnétique nucléaire (Solvant DMSO $D_6$)
δ : 1,45 ppm, singulet, 18H (2.C($CH_3$)$_3$)
δ : 5,62 ppm, signal à allure de singulet, 1H, OH
δ : 9,77 ppm, signal à allure de singulet, 1H, NH

**EXEMPLE 3 : 6-(3',5'-DITERT.BUTYL 4'-HYDROXY CINNAMOYL) BENZOTHIAZOLINONE**

En procédant comme dans l'exemple 2, mais en remplaçant la 5-acétyl benzoxazolinone par la 6-acétyl benzothiazolinone, on obtient le produit du titre.

Solvant de recristallisation : acétonitrile
Rendement : 65%
Point de fusion : 262-264 ° C

| Analyse élémentaire : | | | | |
|---|---|---|---|---|
| | C | H | N | O |
| Calculée :<br>Trouvée : | 70,38<br>70,42 | 6,65<br>6,68 | 3,42<br>3,25 | 11,72<br>11,94 |

Caractéristiques spectrales :
Infrarouge :
3600 $cm^{-1}$ : ν OH
3150 $cm^{-1}$ : ν NH
1700 $cm^{-1}$ : ν CO (S-CO-N)
1645 $cm^{-1}$ : ν CO (cétonique)
Résonance magnétique nucléaire (Solvant $CDCl_3$) :
δ : 2,10 ppm, singulet, 18H (2.C($CH_3$)$_3$)
δ : 5,60 ppm, singulet, 1H, OH
δ : 7,32 ppm, doublet, 1H, COC<u>H</u>, J = 16Hz
δ : 7,85 ppm, doublet, 1H, COCH=C<u>H</u>, J = 16Hz

**EXEMPLE 4 : 6-(3',5'-DITERT.BUTYL 4'-HYDROXY CINNAMOYL) 3-METHYL BENZOTHIAZOLINONE**

En procédant comme dans l'exemple 1 et en remplaçant la 5-acétyl 3-méthyl benzoxazolinone par la 6-acétyl 3-méthyl benzothiazolinone, on obtient le produit du titre.
Solvant de recristallisation : acétone
Rendement : 77%
Point de fusion : 230-232 ° C

| Analyse élémentaire : | | | |
|---|---|---|---|
| | C | H | N |
| Calculée : | 70,88 | 6,90 | 3,31 |
| Trouvée : | 71,01 | 6,90 | 3,20 |

Caractéristiques spectrales :
Infrarouge :
3520 $cm^{-1}$ : $\nu$ OH
1690 $cm^{-1}$ : $\nu$ CO (NCOS)
1650 $cm^{-1}$ : $\nu$ CO (cétonique)
Résonance magnétique nucléaire (Solvant $CDCl_3$) :
$\delta$ : 1,50 ppm, singulet, 18H ($2.C(CH_3)_3$)
$\delta$ : 3,50 ppm, singulet, 3H, N-$CH_3$
$\delta$ : 7,35 ppm, doublet, 1H, CO-C<u>H</u>, J = 15,7Hz
$\delta$ : 7,85 ppm, doublet, 1H, CO-CH=C<u>H</u>, J = 15,7Hz

**EXEMPLE 5 : 6-(3',5'-DITERT.BUTYL 4'-HYDROXY CINNAMOYL)3-METHYL BENZOXAZOLINONE**

Procéder comme dans l'exemple 1 mais en remplaçant la 5-acétyl-3-méthyl benzoxazolinone par la 6-acétyl 3-méthyl benzoxazolinone, on obtient le produit du titre.
Solvant de recristallisation : acétone
Point de fusion : 210-215 ° C

| Analyse élémentaire : | | | |
|---|---|---|---|
| | C | H | N |
| Calculée : | 73,68 | 7,17 | 3,43 |
| Trouvée : | 73,87 | 7,15 | 3,40 |

Caractéristiques spectrales :
conformes à la structure

**EXEMPLE 6 : 6-(3',5'-DITERT.BUTYL 4'-HYDROXY CINNAMOYL) BENZOXAZOLINONE**

Procéder comme dans l'exemple 2 mais en remplaçant la 5-acétyl benzoxazolinone par la 6-acétyl benzoxazolinone, on obtient le produit du titre.
Point de fusion : 215 ° C
Caractéristiques spectrales :
conformes à la structure

**EXEMPLE 7 : 7-(3',5'-DITERT.BUTYL 4'-HYDROXY CINNAMOYL) [1,4]BENZOXAZIN-3-ONE**

En procédant comme dans l'exemple 2, mais en remplaçant la 5-acétyl benzoxazolinone par la 7-acétyl-[1,4]-benzoxazin-3-one, on obtient le produit du titre.
Solvant de recristallisation : méthanol
Point de fusion : 222 ° C

| Analyse élémentaire : | | | | |
|---|---|---|---|---|
| | C | H | N | O |
| Calculée :<br>Trouvée : | 73,68<br>73,17 | 7,17<br>7,34 | 3,43<br>3,39 | 15,70<br>16,00 |

Caractéristiques spectrales :
conformes à la structure

**EXEMPLE 8 : 4-METHYL 7-(3',5'-DITERT.BUTYL 4'-HYDROXY CINNAMOYL) [1,4]BENZOXZIN-3-ONE**

En procédant comme dans l'exemple 1, mais en remplaçant la 5-acétyl 3-méthyl benzoxazolinone par la 7-acétyl 3-méthyl-[1,4]-benzoxazinone, on obtient le produit du titre.
Solvant de recristallisation : méthanol
Point de fusion : 174-176 ° C

| Analyse élémentaire : | | | |
|---|---|---|---|
| | C | H | N |
| Calculée :<br>Trouvée : | 74,08<br>73,82 | 7,41<br>7,51 | 3,32<br>3,39 |

Caractéristiques spectrales :
conformes à la structure

En procédant comme dans les exemples précédents et en remplaçant le 3,5-ditert.butyl 4-hydroxy benzaldéhyde par le 3,4,5-triméthoxy benzaldéhyde, on obtient respectivement :

**EXEMPLE 9 : 5-(3',4',5'-TRIMETHOXY CINNAMOYL)-3-METHYL BENZOXAZOLINONE**

**EXEMPLE 10 : 5-(3',4',5'-TRIMETHOXY CINNAMOYL) BENZOXAZOLINONE**

**EXEMPLE 11 : 6-(3',4',5'-TRIMETHOXY CINNAMOYL) BENZOXAZOLINONE**

**EXEMPLE 12 : 6-(3',4',5'-TRIMETHOXY CINNAMOYL)-3-METHYL BENZOXAZOLINONE**

**EXEMPLE 13 : 6-(3',4',5'-TRIMETHOXY CINNAMOYL) 3-METHYL BENZOXAZOLINONE**

**EXEMPLE 14 : 6-(3',4',5'-TRIMETHOXY CINNAMOYL) BENZOXAZOLINONE**

**EXEMPLE 15 : 7-(3',4',5'-TRIMETHOXY CINNAMOYL) [1,4]-BENZOXAZIN-3-ONE**

**EXEMPLE 16 : 4-METHYL 7-(3',4',5'-TRIMETHOXY CINNAMOYL)-[1,4]-BENZOXAZIN -3-ONE**

En remplaçant dans les exemples 1 et 9 la 5-acétyl-3-méthyl benzoxazolinone par la :
7-acétyl [2,4]-benzoxazin-3-one, on obtient :

**EXEMPLE 17 : 7-(3',5'-DITERT.BUTYL 4'-HYDROXY CINNAMOYL)[2,4] BENZOXAZIN -3-ONE**

**EXEMPLE 18 : 7-(3',4'5'-TRIMETHOXY CINNAMOYL)[2,4]-BENZOXAZIN-3-ONE**

En remplaçant dans les exemples 1 et 9, la 5-acétyl-3-méthyl benzoxazolinone par la :
7-acétyl [2,4]-benzothiazin-3-one, on obtient :

**EXEMPLE 19 : 7-(3',5'-DITERT.BUTYL 4'-HYDROXY CINNAMOYL)[2,4]-BENZOTHIAZIN-3-ONE**

**EXEMPLE 20 : 7-(3',4'5'-TRIMETHOXY CINNAMOYL)[2,4]-BENZOTHIAZIN-3-ONE**

En remplaçant dans les exemples 1 et 9 la 5-acétyl 3-méthyl benzoxazolinone par la :
7-acétyl [1,4]-benzothiazin-3-one décrite dans Ann. Chim. (Rome) 1955, 45, 1972, on obtient :

**EXEMPLE 21 : 7-(3',5'-DITERT.BUTYL 4'-HYDROXY CINNAMOYL)[1,4] BENZOTHIAZIN-3-ONE**

**EXEMPLE 22 : 7-(3',4'5'-TRIMETHOXY CINNAMOYL)[1,4]-BENZOTHIAZIN-3-ONE**

En remplaçant dans les exemples 1 et 9 la 5-acétyl 3-méthyl benzoxazolinone par la :
7-acétyl 2-méthyl [1,4]-benzoxazin-3-one, on obtient :

**EXEMPLE 23 : 7-(3',5'-DITERT.BUTYL 4'-HYDROXY CINNAMOYL) 2-METHYL [1,4]-BENZOXAZIN-3-ONE**

**EXEMPLE 24 : 7-(3',4',5'-TRIMETHOXY CINNAMOYL) 2-METHYL [1,4]-BENZOXAZIN -3-ONE**

En remplaçant dans les exemples 1 et 9 la 5-acétyl 3-méthyl benzoxazolinone par la :
7-acétyl 2,2-diméthyl [1,4]-benzoxazin-3-one, on obtient :

**EXEMPLE 25 : 7-(3',5'-DITERT.BUTYL 4'-HYDROXY CINNAMOYL) 2,2-DIMETHYL [1,4]-BENZOXAZIN-3-ONE**

**EXEMPLE 26 : 7-(3',4',5'-TRIMETHOXY CINNAMOYL) 2,2-DIMETHYL [1,4]-BENZOXAZIN-3-ONE**

**EXEMPLE 27 : 2,3-DIHYDRO 3,5-DIMETHYL 6-(3',5'-DITERT.BUTYL 4'-HYDROXY BENZYLIDENE) CYCLOPENTA [f] BENZOXAZOL-2,7-DIONE**

En procédant comme dans l'exemple 1, mais en remplaçant la 5-acétyl 3-méthyl benzoxazolinone par la 2,3-dihydro 3,5-diméthyl cyclopenta [f] benzoxazol-2,7-dione décrite dans la demande de brevet France 90.07812, on obtient le produit du titre.
Solvant de recristallisation : toluène
Point de fusion : 217-218 ° C

**EXEMPLE 28 : 2,3-DIHYDRO 3,8-DIMETHYL 6-(3',5'-DITERT.BUTYL 4'-HYDROXY BENZYLIDENE) CYCLOHEXA [f] BENZOXAZOL-2,5-DIONE**

En procédant comme dans l'exemple 1, mais en remplaçant la 5-acétyl 3-méthyl benzoxazolinone par la 2,3-dihydro 3,8-diméthyl cyclohexa [f] benzoxazol-2,5-dione décrite dans la demande de brevet France 90.07812, on obtient le produit du titre.
Solvant de recristallisation : éthanol 90
Point de fusion : 228-229 ° C

**EXEMPLE 29 : 2,3-DIHYDRO 3,7-DIMETHYL 6-(3',5'-DITERT.BUTYL 4'-HYDROXY BENZYLIDENE) CYCLOPENTA [f] BENZOXAZOL-2,5-DIONE**

En procédant comme dans l'exemple 1, mais en remplaçant la 5-acétyl 3-méthyl benzoxazolinone par la 2,3-dihydro 3,7-diméthyl cyclopenta [f] benzoxazol-2,5-dione décrite dans la demande de brevet France 90.07812, on obtient le produit du titre.
Solvant de recristallisation : toluène
Point de fusion : > 270 ° C

**EXEMPLES 30 A 32 :**

En procédant comme dans les exemples 27 à 29, mais en remplaçant le 3,5-ditert.butyl 4-hydroxy benzaldéhyde par le 3,4,5-triméthoxy benzaldéhyde, on obtient :

**EXEMPLE 30 : 2,3-DIHYDRO 3,5-DIMETHYL 6-(3',4',5'-TRIMETHOXY BENZYLIDENE) CYCLOPENTA [f] BENZOXAZOL-2,7-DIONE**

**EXEMPLE 31 : 2,3-DIHYDRO 3,8-DIMETHYL 6-(3',4',5'-TRIMETHOXY BENZYLIDENE) CYCLOHEXA [f] BENZOXAZOL-2,5-DIONE**

**EXEMPLE 32 : 2,3-DIHYDRO 3,7-DIMETHYL 6-(3',4',5'-TRIMETHOXY BENZYLIDENE) CYCLOPENTA [f] BENZOXAZOL-2,5-DIONE**

**EXEMPLE 33 : 2,3-DIHYDRO 5-METHYL 6-(3',5'-DITERT.BUTYL 4'-HYDROXY BENZYLIDENE) CYCLOPENTA [f] BENZOXAZOL-2,7-DIONE**

En procédant comme dans l'exemple 1, mais en remplaçant la 5-acétyl 3-méthyl benzoxazolinone par la 2,3-dihydro 5-méthyl cyclopenta [f] benzoxazol 2,7-dione, on obtient le produit du titre.
Point de fusion : supérieur à 260 ° C

**EXEMPLE 34 : 2,3-DIHYDRO 8-METHYL 6-(3',5'-DITERT.BUTYL 4'-HYDROXY BENZYLIDENE) CYCLOHEXA [f] BENZOXAZOL 2,5-DIONE**

En procédant comme dans l'exemple 1, mais en remplaçant la 5-acétyl 3-méthyl benzoxazolinone par la 2,3-dihydro 8-méthyl cyclohexa [f] benzoxazol 2,5-dione décrite dans la demande de brevet Fr 90.07812, on obtient le produit du titre.
Point de fusion : 228-229 ° C

**EXEMPLE 35 : 2,3-DIHYDRO 5-PHEHYL 6-3',5'-DITERT.BUTYL 4'-HYDROXY BENZYLIDENE) CYCLOPENTA [f] BENZOXAZOL 2,7-DIONE**

En procédant comme dans l'exemple 1, mais en remplaçant la 5-acétyl 3-méthyl benzoxazolinone par la 2,3-dihydro 5-phényl cyclopenta [f] benzoxazol 2,5-dione obtenue dans la préparation 1, on obtient le produit du titre.
Temps de réaction : 15 heures
Solvant de recristallisation : toluène
Point de fusion : supérieur à 260 ° C
Caractéristiques spectrales : RMN $^1H$
$\delta$ = 1,28 ppm, singulet 18H, 2-$C(CH_3)_3$

**EXEMPLE 36 : 2,3-DIHYDRO 3-METHYL 5-PHENYL 6-(3',5'-DITERT.BUTYL 4'-HYDROXY BENZYLIDENE) CYCLOPENTA [f] BENZOXAZOL 2,7-DIONE**

En procédant comme dans l'exemple 1, mais en remplaçant la 5-acétyl 3-méthyl benzoxazolinone par la 2,3-dihydro 3-méthyl 5-phényl cyclopenta [f] benzoxazol 2,7-dione obtenue dans la préparation 2, on obtient le produit du titre.
Temps de réaction : 15 heures
Solvant de recristallisation : toluène cyclohexane (1/1)
Point de fusion : 229-230 ° C
Caractéristiques spectrales : RMN $^1H$
$\delta$ = 1,33 ppm, singulet 18H, 2-$C(CH_3)_3$

**EXEMPLE 37 : 6-(3',5'-DITERT.BUTYL 4'-HYDROXY CINNAMOYL)1,4-BENZOTHIAZIN -3-ONE**

En procédant comme dans l'exemple 1, mais en remplaçant la 5-acétyl 3-méthyl benzoxazolinone par la 6-acétyl 1,4-benzothiazin-3-one décrite dans Indian J. Chem. 1983,22B, 1236, on obtient le produit du titre.

**EXEMPLE 38 : 8-(3',5'-DITERT.BUTYL 4'-HYDROXY CINNAMOYL)1,4-BENZOTHIAZIN -3-ONE**

En procédant comme dans l'exemple 1, mais en remplaçant la 5-acétyl 3-méthyl benzoxazolinone par la 8-acétyl 1,4-benzothiazin-3-one décrite dans la publication Eur. J. Med. Chem., 89, 24, 5, 483, on obtient

le produit du titre.

**EXEMPLE 39 : 2,3-DIHYDRO 3-METHYL 5-ETHYL 6-(3',5'-DITERT.BUTYL 4'-HYDROXY BENZYLIDENE) CYCLOPENTA [f] BENZOXAZOL-2,7-DIONE**

En procédant comme dans l'exemple 1, mais en remplaçant la 5-acétyl 3-méthyl benzoxazolinone par la 2,3-dihydro 2,7-dioxo 5-éthyl cyclopenta [f] benzoxazol 2,7-dione, on obtient le produit du titre.

**EXEMPLE 40 : 2,3-DIHYDRO 3-METHYL 2,7-DIOXO 6-(3',5'-DITERT.BUTYL 4'-HYDROXY BENZYLIDENE) CYCLOPENTA [f] BENZOXAZOL-2,7-DIONE**

En procédant comme dans l'exemple 1, mais en remplaçant la 5-acétyl 3-méthyl benzoxazolinone par le 2,3-dihydro 2,7-dioxo 3-méthyl cyclopenta [f] benzoxazole obtenu dans la préparation 6, on obtient le produit du titre.

**EXEMPLE 41 : [4H] 2,3-DIHYDRO 3,8-DIOXO 4-METHYL 6-PHENYL 7-(3',5'-DITERT.BUTYL 4'-HYDROXY BENZYLIDENE) CYCLOPENTA [g] 1,4-BENZOXAZINE**

En procédant comme dans l'exemple 1, mais en remplaçant la 5-acétyl 3-méthyl benzoxazolinone par le [4H] 2,3-dihydro 3,8-dioxo 4-méthyl 6-phényl cyclopenta [g] 1,4-benzoxazole obtenu dans la préparation 7, on obtient le produit du titre.

**EXEMPLE 42 : [4H] 2,3-DIHYDRO 3,8-DIOXO 4-METHYL 6-PHENYL 7-(3',5'-DITERT.BUTYL 4'-HYDROXY BENZYLIDENE) CYCLOPENTA [g] 1,4-BENZOTHIAZINE**

En procédant comme dans l'exemple 1, mais en remplaçant la 5-acétyl 3-méthyl benzoxazolinone par la [4H] 2,3-dihydro 3,8-dioxo 4-méthyl 6-phényl cyclopenta [g] 1,4-benzothiazine obtenue dans la préparation 11, on obtient le produit du titre.

**EXEMPLE 43 : [4H] 2,3-DIHYDRO 3,8-DIOXO 2-BENZYL 4-METHYL 6-PHENYL 7-(3',5'-DITERT.BUTYL 4'-HYDROXY BENZYLIDENE) CYCLOPENTA [g] 1,4-BENZOXAZINE**

En procédant corne dans l'exemple 1, mais en remplaçant la 5-acétyl 3-méthyl benzoxazolinone par la [4H] 2,3-dihydro 3,8-dioxo 4-méthyl 2-benzyl 6-phényl cyclopenta [f] 1,4-benzoxazine obtenue dans la préparation 10, on obtient le produit du titre.

En procédant comme dans les exemples précédents et en utilisant les matières premières appropriées que l'homme de métier obtiendra sans difficulté à partir des modes opératoires et de la littérature ci-dessus cités, on obtient :

**[4H] 2,3-DIHYDRO 3,8-DIOXO 4-METHYL 6-THIENYL 7-(3',5'-DITERT.BUTYL 4'-HYDROXY BENZYLIDENE) CYCLOPENTA [g] 1,4-BENZOXAZINE**

**[3H] 2,3-DIHYDRO 2,7-DIOXO 3-METHYL 5-THIENYL 6-(3',5'-DITERT.BUTYL 4'-HYDROXY BENZYLIDENE) CYCLOPENTA [f] 1,4-BENZOXAZOLE**

**[3H] 2,3-DIHYDRO 2,7-DIOXO 3-METHYL 5-(3',5'-DITERT.BUTYL 4'-HYDROXY PHENYL) 6-(3',5'-DITERT.BUTYL 4'-HYDROXY BENZYLIDENE) CYCLOPENTA [g] 1,3-BENZOXAZOLE**

**[4H] 2,3-DIHYDRO 3,9-DIOXO 4,7- DIMETHYL 8-(3',5'-DITERT.BUTYL 4'-HYDROXY BENZYLIDENE) CYCLOPENTA [h] 1,4-BENZOXAZINE**

**[4H] 2,3-DIHYDRO 3,8-DIOXO 2,2,4,6-TETRAMETHYL 7-(3',5'-DITERT.BUTYL 4'-HYDROXY BENZYLIDENE) CYCLOPENTA [g] 1,4-BENZOXAZINE**

**[4H] 2,3-DIHYDRO 3,8-DIOXO 2,2,4,6-TETRAMETHYL 7-(3',5'-DITERT.BUTYL 4'-HYDROXY BENZYLIDENE) CYCLOPENTA [g] 1,4-BENZOTHIAZINE**

**[4H] 2,3-DIHYDRO 3,8-DIOXO 2,2,4-TRIMETHYL 6 PHENYL 7-(3',5'-DITERT.BUTYL 4'-HYDROXY BENZYLIDENE) CYCLOPENTA [g] 1,4-BENZOTHIAZINE**

**[4H] 2,3-DIHYDRO 3,8-DIOXO 2,2,4-TRIMETHYL 6-(3',5'-DITERT.BUTYL 4'-HYDROXY PHENYL) 7-(3',5'-DITERT.BUTYL 4'-HYDROXY BENZYLIDENE)CYCLOPENTA [g] 1,4-BENZOTHIAZINE**

**[4H] 2,3-DIHYDRO 3,8-DIOXO 2,2,4,6-TETRAMETHYL 7-(3',5'-DITERT.BUTYL 4'-HYDROXY BENZYLIDENE)CYCLOPENTA [g] 1,4-BENZOTHIAZINE**

**[3H] 2,3-DIHYDRO 5-PHEHYL 6-(3',5'-DITERT.BUTYL 4'-HYDROXY BENZYLIDENE)CYCLOPENTA [f] BENZOTHIAZOL-2,7-DIONE**

**ETUDE PHARMACOLOGIOUE DES DERIVES DE L'INVENTION**

**EXEMPLE A : ETUDE DE LA TOXICITE AIGUE**

La toxicité aiguë a été appréciée après administration orale à des lots de 5 souris (20 ± 2 grammes) de doses croissantes (0,1 - 0,25 - 0,50 - 0,75 - 1 g/kg). Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose 1 $g.kg^{-1}$. On ne constate pas de troubles après administration de cette dose.

**EXEMPLE B : MISE EN EVIDENCE D'UNE ACTIVITE ANTIPEROXYDANTE**

L'étude de la capacité des composés de l'invention à piéger les radicaux libres OH• a été étudiée sur des homogénats de cerveaux de rats

- d'une part sur la péroxydation spontanée des lipides,
- d'autre part sur la péroxydation induite par le système $Fe^{++}$ -Ascorbate (10 $\mu$M - 250 $\mu$M)

a/ Mesure de l'inhibition de la péroxydation lipidique spontanée

Les homogénats de cerveaux de rats sont placés en présence ou en absence des composés à tester durant 60 minutes à 37°C. L'intensité de la péroxydation lipidique en présence et en absence des dérivés de l'invention est déterminée par les substances réagissant avec l'acide thiobarbiturique exprimées en mmoles de Malondialdéhyde par une méthode dérivée de celle de YAGI (K. YAGI, Biochem. Med. 1976, 15, 212-216).

b/ Mesure de l'inhibition de la péroxydation lipidique induite

La méthodologie est identique à l'exception de l'addition à l'homogénat du système inducteur de radicaux $Fe^{++}$ - Ascorbate.

Les substances de référence sont dans les deux cas le probucol et la vitamine E. Certains composés de l'invention provoquent dans un test une inhibition de la lipopéroxydation supérieure à celle du Probucol, le composé commercialisé le plus actif dans ce domaine d'activité et à celle de la vitamine E réputée pour ses propriétés antioxydantes. Ainsi, les exemples 33, 34, 35, 36, 40 entraînent à une concentration de $10^{-5}$M une inhibition de la péroxydation spontanée d'environ 50%. Une concentration identique de Probucol entraîne une inhibition de 37%. Une concentration identique de vitamine E une inhibition de 18%.

En ce qui concerne le test de la péroxydation induite, les composés des exemples 33, 34, 35, 36 à une concentration de $10^{-5}$M entraînent une inhibition voisine de 50%. Celui de l'exemple 4 une inhibition voisine de 30%. A titre de comparaison l'inhibition provoquée dans ce test par une concentration $10^{-5}$M de Probucol est de 27% et par une concentration $10^{-5}$M de vitamine E de 6%.

Le composé MZ130 de la demande de brevet EP 0223674, l'exemple 1 et le composé 6 du tableau 1 de la demande de brevet Fr 73.23281 n'ont pas révélé d'activité significative sur ces tests.

**EXEMPLE C : MESURE DE L'INHIBITION DE FORMATION DE DIENES CONJUGUES PAR ACTIVITE RADICALAIRE**

Cette méthodologie consiste à générer des radicaux libres OH- au sein d'une émulsion d'acide linoléique grâce à un système constitué par un mélange $Fe^{2+}/Fe^{3+}$. Les composés de l'invention inhibent de façon intense la formation des diènes. A une concentration $10^{-5}$M les dérivés des exemples 2, 6, 7 inhibent d'environ 75% la formation des diènes.

Le dérivé de l'exemple 36 inhibe d'environ 50% la formation des diènes.

A titre de comparaison à une concentration de $10^{-5}$M le Probucol n'inhibe cette formation que de 24% et le dérivé MZ 130 de la demande EP 0223674, l'exemple 1 et le composé 6 du tableau 1 de la demande Fr 73.23281 n'ont pas d'action significative sur ce test.

**EXEMPLE D : MESURE DU NIVEAU D' OXYDATION DES LDL**

Dans ces conditions les radicaux libres sont générés par du Cu + + au sein d'un milieu contenant des LDL natives en présence ou absence des composés à étudier. La réaction est arrêtée par adjonction au milieu réactionnel de 2,6-ditert.butyl 4-méthyl phénol et d'E.D.T.A. et les produits d'oxydation sont dosés par FPLC.

Chaque préparation de LDL est testée avec le Probucol comme référence dont l'activité est cotée par l'indice de péroxydation i = 1.

Les résultats des produits étudiés sont donc exprimés par rapport à cette activité du probucol :

$$ix = \frac{Dx - Dtémoin}{DProbucol - Dtémoin}$$

D étant la hauteur du pic de la fraction 27/28 du chromatogramme, la somme des fractions oxydées 27/28 et 31/32 est égale à 100.

Pour les produits très actifs, la fraction oxydée 27/28 disparaît. C'est ce que l'on observe avec les produits des exemples 27, 28, 29, 33, 34, 35, 36.

A titre de comparaison le produit MZ130 de la demande EP 0223674, l'exemple 1 et le composé 6 du tableau 1 de la demande Fr 73.23281 n'ont pas d'activité significative sur ce test.

**EXEMPLE E : MESURE DE L'HEMOLYSE INDUITE PAR L'AAPH**

Des hématies humaines sont placées en présence d'AAPH (chlorhydrate de 2,2' azo bis(2-amidino propane), générateur de radicaux libres à taux constant et en présence ou absence des composés à étudier durant 30 minutes à 37°C. La densité optique du surnageant est mesurée à 403 nm par rapport au témoin sans AAPH. Le pourcentage d'inhibition de l'hémolyse est calculé par comparaison au 100% d'hémolyse obtenu pour le témoin AAPH.

Les composés de l'invention se sont montrés capables d'inhiber fortement l'hémolyse induite par l'AAPH.

Ainsi, les composés des exemples 2, 3, 5, 6 inhibent entre 78 et 88% l'hémolyse induite par l'AAPH à une concentration de $10^{-5}$M. A titre de comparaison, le Probucol à une concentration de $10^{-5}$M ne provoque qu'une inhibition de 48%. Le produit MZ130 de la demande EP 0223674, l'exemple 1 et le composé 6 du tableau 1 de la demande Fr 73.23281 n'ont pas d'activité significative sur ce test.

**EXEMPLE F : MESURE DE L'INHIBITION DE L'ACTIVITE DE LA LIPOXYGENASE**

L'activité de la lipoxygénase est mesurée à partir de polynucléaires humains lavés, en présence et absence des dérivés revendiqués après activation par le calcium (A 23/87). Le leukotriène B4 (LTB4) produit est mesuré par radioimmunoassay (Gresele, P. et Coll. Biochem. Biophys. Res. Comm. 1986, 137, 334-342).

Ainsi, à une concentration de 10-5M l'exemple 1 inhibe d'environ 80% l'action de la lipoxygénase, les exemples 5, 35 et 36 d'environ 60% alors que le Probucol utilisé comme référence à une concentration de $10^{-5}$M n'entraîne qu'une inhibition de 44%.

**EXEMPLE G : ETUDE DE L'ACTIVITE HYPOGLYCEMIANTE**

Des souris mâles KK ont été placées dans des cages à l'âge de huit semaines. Elles sont utilisées pour l'expérience lorsque leur poids est supérieur à 40 grammes à l'âge de 4-5 mois.

Le composé de l'invention est placé en suspension dans du sirop de gomme. Chaque composé testé est administré oralement à une dose de 150 ou 50 mg/kg, 18 heures avant le prélèvement sanguin. Le sang est recueilli par prélèvement au niveau de la veine caudale dans un tube à hématocrite, puis centrifugé. Le plasma est recueilli et le dosage de la glycémie effectué.

Il apparaît que les composés de l'invention diminuent la glycémie de façon significative.

**COMPOSITION PHARMACEUTIOUE**

**EXEMPLE : COMPRIMES**

Comprimés dosés à 20 mg de [3H] 2,3-dihydro 3-méthyl 5-phényl 6-(3',5'-ditert.butyl 4'-hydroxy benzylidène) cyclopenta [f] benzoxazol 2,7-dione

| Formule de préparation pour 1000 comprimés : | |
|---|---|
| [3H] 2,3-dihydro 3-méthyl 5-phényl 6-(3',5'-ditert.butyl 4'-hydroxy benzylidène) cyclopenta [f] benzoxazol 2,7-dione | 20 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

**Revendications**
**Revendications pour les Etats contractants suivants : AT, BE, CH, DE, DK, FR, GB, IT, LI, LU, NL, SE**

**1.** Composés de formule (I) :

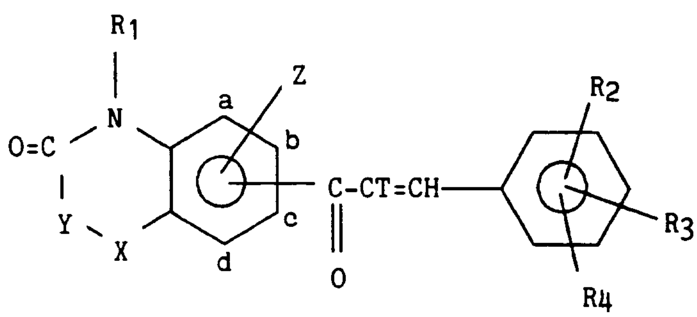

(I)

dans laquelle :

$R_1$ représente un atome d'hydrogène ou un radical alkyle inférieur,

X représente :

. un atome d'oxygène ou de soufre,

. un groupement $CH_2$ à la condition que dans ce cas Y représente un atome d'oxygène ou de soufre,

Y représente :

. une liaison simple ou un groupement $CR_7R_8$ où $R_7$ et $R_8$ identiques ou différents représentent indépendamment l'un de l'autre un atome d'hydrogène, un groupement alkyle inférieur, un groupement phényle, un groupement phényle substitué, un groupement phényl alkyle ou un groupement phényl alkyle substitué,

. un atome d'oxygène ou de soufre à la condition que dans ce cas X représente un groupement $CH_2$,

Z représente ou bien un atome d'hydrogène et dans ce cas T représente également un atome d'hydrogène ou bien Z forme avec T un chaînon $-(CH_2)_n-CH(E)-$ ou (CH2)n-CH($CH_2$E') avec n entier égal à 0, 1, 2 ou 3 étant entendu que dans ce cas Z est porté par un carbone voisin du carbone porteur du groupement acylé, et E ou E' représente un atome d'hydrogène, un groupement alkyle inférieur, un groupement phényle, hétéroaryle, ou phényle substitué ou hétéroaryle substitué,

le terme substitué affectant les termes phényle et hétéroaryle et phényle alkyle dans la définition de $R_7$ et $R_8$ et E et E' signifiant que ces groupements peuvent être substitués par un ou plusieurs substituants choisis parmi alkyle inférieur, alkoxy inférieur, halogène, trifluorométhyle ou hydroxyle,

$R_2$, $R_3$ et $R_4$ identiques ou différents représentent indépendamment l'un de l'autre un groupement hydroxy ou alkoxy inférieur ou un groupement alkyle inférieur aux conditions que :

. lorsque X représente l'oxygène, Y représente une liaison simple et Z représente un atome d'hydrogène, l'un au moins de $R_2$, $R_3$ et $R_4$ est différent du groupement méthoxy,

. lorsque X représente l'oxygène, Y représente un groupement $CR_7R_8$ et Z représente un atome d'hydrogène, l'un au moins de $R_2$, $R_3$, $R_4$ n'est pas un groupement alkyle,

leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable,

étant entendu que par alkyle inférieur ou alkoxy inférieur, on entend des groupements linéaires ou ramifiés comprenant de 1 à 6 atomes de carbone,

que par hétéroaryle on entend des groupements non saturés mono ou bicycliques comprenant de 5 à 12 atomes autres que l'hydrogène et incluant dans un squelette carboné de 1 à 3 hétéroatomes choisis parmi azote, oxygène ou soufre.

**2.** Composés selon la revendication 1 pour lesquels $R_3$ représente le groupement hydroxyle et $R_2$ et $R_4$ représentent chacun simultanément un groupement tert.butyl, leurs isomères et leurs sels d'addition à une base pharmaceutiquement acceptable.

**3.** Composés selon la revendication 1 pour lesquels Z forme avec T un chaînon $(CH_2)_n-CH(E)$ ou $(CH_2)_n-CH(CH_2E')$ avec n entier égal à 0, 1, 2 ou 3 étant entendu que dans ce cas Z est porté par un carbone voisin du carbone porteur du groupement acylé, et E ou E' représente un atome d'hydrogène, un groupement alkyle inférieur, un groupement phényle, hétéroaryle, ou phényle substitué ou hétéroaryle substitué,
leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable.

**4.** Composés selon la revendication 1 pour lesquels Z et T représentent simultanément un atome d'hydrogène, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

**5.** Composés selon la revendication pour lesquel Y représente une liaison simple ou un groupement $CH_2$, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

**6.** Composés selon la revendication 1 pour lesquels X représente un atome d'oxygène ou de soufre, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

7. Composés selon la revendication 3 pour lequel E représente un groupement phényle éventuellement substitué par un ou plusieurs groupements choisis parmi hydroxyle et alkyle inférieur, leurs isomères et leurs sels d'addition à une base pharmaceutiquement acceptable.

8. Composé selon la revendication 1 qui est la 7-(3',5' ditert.butyl 4'-hydroxy cinnamoyl) [1,4]benzothiazin-3-one, ses isomères et ses sels d'addition à une base pharmaceutiquement acceptable.

9. Composés selon la revendication 1 qui est la 6-(3',5'-ditert.butyl 4'-hydroxy cinnamoyl) benzoxazolinone, ses isomères et ses sels d'addition à une base pharmaceutiquement acceptable.

10. Composé selon la revendication 1 qui est la 2,3-dihydro 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

11. Composé selon la revendication 1 qui est la [4H] 2,3-dihydro 3,8-dioxo 2,2,4-triméthyl 6-(3',5'-ditert.butyl 4'-hydroxy phényl) 7-(3',5'-ditert.butyl 4'-hydroxybenzilidène) cyclopenta [g] 1,4-benzothiazine, ses isomères et ses sels d'addition à une base pharmaceutiquement acceptable.

12. Composé selon la revendication 1 qui est [4H] 2,3-dihydro 3,8-dioxo 4-méthyl 6-phényl 7-(3',5'-ditert.butyl 4'-hydroxy benzylidène) cyclopenta [g] 1,4-benzothiazine, ses isomères et ses sels d'addition à une base pharmaceutiquement acceptable.

13. Composé selon la revendication 1 qui est la 2,3-dihydro 3-méthyl 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

14. Composé selon la revendication 1 qui est la 2,3-dihydro 5-phényl 6-(3',5' ditert.butyl 4'-hydroxy benzylidène) cyclopenta [f] benzothiazol 2,7-dione, ses isomères et ses sels d'addition à une base pharmaceutiquement acceptable.

15. Procédé de préparation des composés de formule (I) selon la revendication 1 caractérisé en ce que l'on fait réagir un composé de formule (II) :

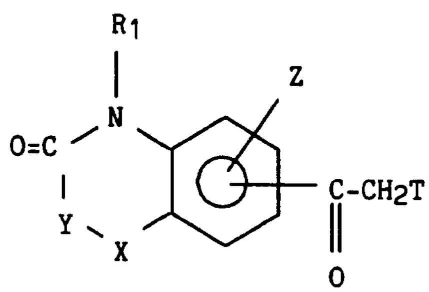

(II)

dans laquelle $R_1$, X, Y, Z et T ont la même signification que dans la formule (I),
en milieu organique acide avec un aldéhyde de formule (III) :

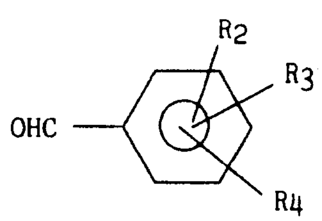

(III)

dans laquelle $R_2$, $R_3$ et $R_4$ ont la même définition que dans la formule (I),
pour conduire, après éventuelle neutralisation du milieu réactionnel, à un dérivé de formule (I), que l'on

purifie si nécessaire par une technique choisie parmi chromatographie ou cristallisation, dont on sépare les isomères, le cas échéant et que l'on salifie, si on le désire, lorsque Ri représente un atome d'hydrogène ou l'un au moins des groupements $R_2$, $R_3$ et $R_4$ représente un groupement hydroxyle, par une base pharmaceutiquement acceptable.

**16.** Composés de formule (II)

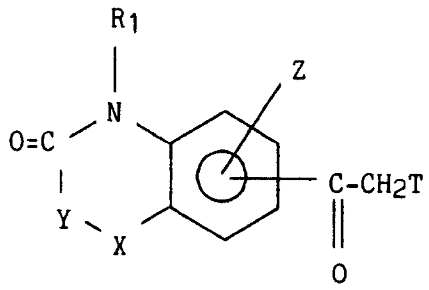

(II)

dans laquelle :

$R_1$ représente un atome d'hydrogène ou un radical alkyle inférieur,

X représente :
- . un atome d'oxygène ou de soufre,
- . un groupement $CH_2$ à la condition que dans ce cas Y représente un atome d'oxygène ou de soufre,

Y représente :
- . une liaison simple ou un groupement $CR_7R_8$ où $R_7$ et $R_8$ identiques ou différents représentent indépendamment l'un de l'autre un atome d'hydrogène, un groupement alkyle inférieur, un groupement phényle, un groupement phényle substitué, un groupement phényl alkyle ou un groupement phényl alkyle substitué,
- . un atome d'oxygène ou de soufre à la condition que dans ce cas X représente un groupement $CH_2$,

Z forme avec T un chaînon $-(CH_2)_n-CH(E)$ ou $-(CH_2)n-CH(CH_2E')$ avec n entier égal à 0, 1, 2 ou 3 étant entendu que dans ce cas Z est porté par un carbone voisin du carbone porteur du groupement acylé, et E ou E' représente un atome d'hydrogène, un groupement alkyle inférieur, un groupement phényle, hétéroaryle, ou phényle substitué ou hétéroaryle substitué, à l'exception des dérivés pour lesquels X représente un atome d'oxygène, Y représente une liaison simple, E représente un groupement méthyle ou E' un atome d'hydrogène ainsi que lorsque $R_1$ représente un atome d'hydrogène ou l'un au moins des groupements $R_2$, $R_3$ et $R_4$ représente un groupement hydroxyle, leurs sels d'addition à une base, utiles en tant qu'intermédiaire de synthèse des composés de formule (I),
le terme substitué affectant les termes phényle et hétéroaryle et phényle alkyle dans la définition de $R_7$ et $R_8$ et E et E' signifiant que ces groupements peuvent être substitués par un ou plusieurs substituants choisis parmi alkyle inférieur, alkoxy inférieur, halogène, trifluorométhyle ou hydroxyle,

**17.** Composition pharmaceutique contenant comme principe actif au moins un composé selon la revendication 1 en combinaison avec un ou plusieurs excipients ou véhicules inertes non toxique pharmaceutiquement acceptable.

**18.** Composition pharmaceutique selon la revendication 17 contenant au moins un principe actif selon l'une des revendications 1 à 15 utilisables dans le traitement de l'inflammation, l'athérosclérose, la prévention et le traitement des troubles plaquettaires dans les hypercholestérolémies, hypertriglycéridémies, dans le diabète non insulino dépendant ainsi que de ses complications

**Revendications pour l'Etat contractant suivant : ES**

**1.** Procédé de préparation de composés de formule (I) ::

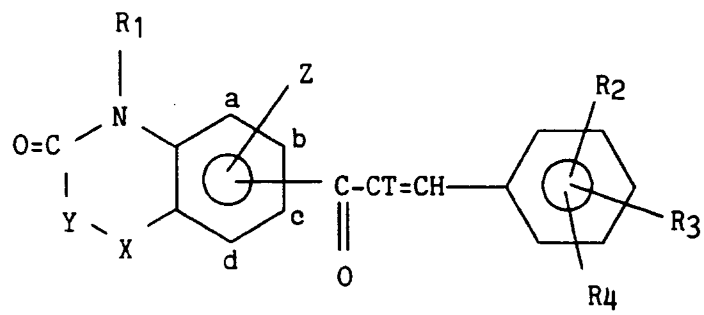

(I)

dans laquelle :

$R_1$ représente un atome d'hydrogène ou un radical alkyle inférieur,

X représente :

. un atome d'oxygène ou de soufre,

. un groupement $CH_2$ à la condition que dans ce cas Y représente un atome d'oxygène ou de soufre,

Y représente :

. une liaison simple ou un groupement $CR_7R_8$ où $R_7$ et $R_8$ identiques ou différents représentent indépendamment l'un de l'autre un atome d'hydrogène, un groupement alkyle inférieur, un groupement phényle, un groupement phényle substitué, un groupement phényl alkyle ou un groupement phényl alkyle substitué,

. un atome d'oxygène ou de soufre à la condition que dans ce cas X représente un groupement $CH_2$,

Z représente ou bien un atome d'hydrogène et dans ce cas T représente également un atome d'hydrogène ou bien Z forme avec T un chaînon $-(CH_2)_n-CH(E)-$ ou $(CH_2)_n-CH(CH_2E')$ avec n entier égal à 0, 1, 2 ou 3 étant entendu que dans ce cas Z est porté par un carbone voisin du carbone porteur du groupement acylé, et E ou E' représentent un atome d'hydrogène, un groupement alkyle inférieur, un groupement phényle, hétéroaryle, ou phényle substitué ou hétéroaryle substitué,

le terme substitué affectant les termes phényle et hétéroaryle et phényle alkyle dans la définition de $R_7$ et $R_8$ et E et E' signifiant que ces groupements peuvent être substitués par un ou plusieurs substituants choisis parmi alkyle inférieur, alkoxy inférieur, halogène, trifluorométhyle ou hydroxyle,

$R_2$, $R_3$ et $R_4$ identiques ou différents représentent indépendamment l'un de l'autre un groupement hydroxy ou alkoxy inférieur ou un groupement alkyle inférieur aux conditions que :

. lorsque X représente l'oxygène, Y représente une liaison simple et Z représente un atome d'hydrogène, l'un au moins de $R_2$, $R_3$ et $R_4$ est différent du groupement méthoxy,

. lorsque X représente l'oxygène, Y représente un groupement $CR_7R_8$ et Z représente un atome d'hydrogène, l'un au moins de $R_2$, $R_3$, $R_4$ n'est pas un groupement alkyle,

leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable,

étant entendu que par alkyle inférieur ou alkoxy inférieur, on entend des groupements linéaires ou ramifiés comprenant de 1 à 6 atomes de carbone,

que par hétéroaryle on entend des groupements non saturés mono ou bicycliques comprenant de 5 à 12 atomes autres que l'hydrogène et incluant dans un squelette carboné de 1 à 3 hétéroatomes choisis parmi azote, oxygène ou soufre,

caractérisé en ce que l'on fait réagir un composé de formule (II) :

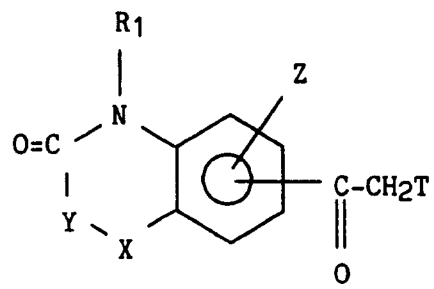

(II)

dans laquelle $R_1$, X, Y, Z et T ont la même signification que dans la formule (I), en milieu organique acide avec un aldéhyde de formule (III) :

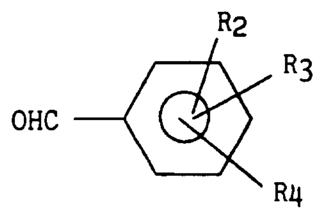

(III)

dans laquelle $R_2$, $R_3$ et $R_4$ ont la même définition que dans la formule (I), pour conduire, après éventuelle neutralisation du milieu réactionnel, à un dérivé de formule (I), que l'on purifie si nécessaire par une technique choisie parmi chromatographie ou cristallisation, dont on sépare les isomères, le cas échéant et que l'on salifie, si on le désire, lorsque $R_1$ représente un atome d'hydrogène ou l'un au moins des groupements $R_2$, $R_3$ et $R_4$ représente un groupement hydroxyle, par une base pharmaceutiquement acceptable.

**2.** Procédé de préparation de composés de formule (II) selon la revendication 1 pour lesquels Z forme avec T un chaînon $-(CH_2)_n-CH(E)$ ou $(CH_2)_n-CH(CH_2E')$ à l'exception des dérivés pour lesquels X représente un atome d'oxygène, Y représente une liaison simple, E représente un groupement méthyle ou E' un atome d'hydrogène,
caractérisé par ce que l'on cyclise en milieu acide un dérivé de formule (IV) :

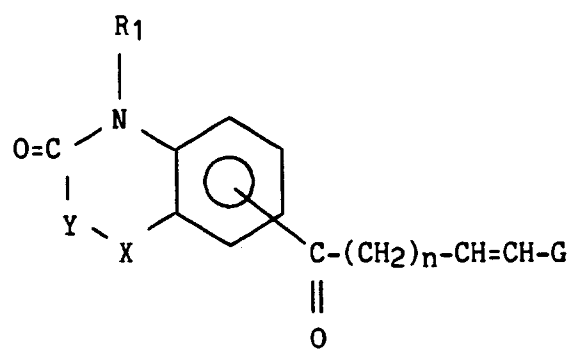

(IV)

avec n, Y, X et $R_1$ ayant la même définition que dans la formule (I) et G représente E ou $CH_2E'$, dérivé de formule (IV) que l'on obtiendra,

- soit lorsque Y représente une liaison simple, par acylation en milieu acide d'un dérivé de formule (V) :

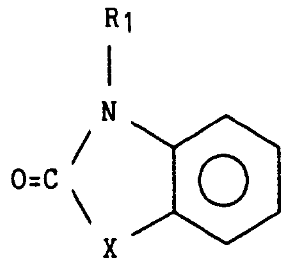

(V)

dans laquelle $R_1$ et X ont la même définition que dans la formule (I), par un chlorure d'acide de formule (VI) :

$ClOC-(CH_2)_nCH=CH-E$ **(VI)**

dans laquelle n et E ont la même définition que dans la formule (I), ou par un dérivé de formule (VII) :

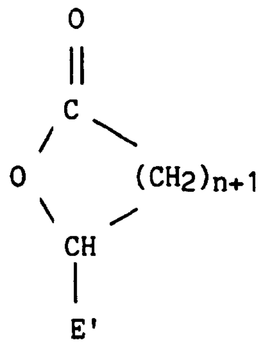

(VII)

dans laquelle n et E' ont la même définition que dans la formule (I),

- soit lorsque n = 0 par condensation d'un dérivé de formule (VIII) :

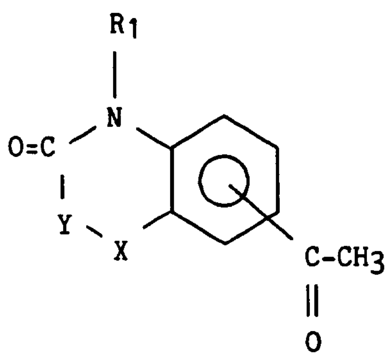

(VIII)

ou X, Y, $R_1$ et n ont la même définition que dans la formule (I), avec un aldéhyde de formule (IX) :

G-CHO **(IX)**

ou G a la même définition que précédemment,

- soit, lorsque G représente un atome d'hydrogène et n = 0, par déhydrohalogénation en milieu alcalin d'un dérivé de formule (X) :

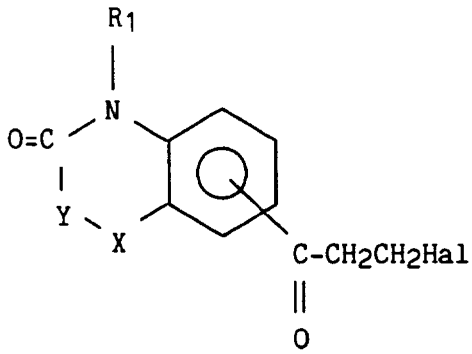

(X)

ou $R_1$, X, Y et n ont la même définition que précédemment et Hal représente un atome d'halogène,

avec le cas échéant purification et/ou séparation des isomères.

**3.** Procédé selon la revendication 1 de préparation de composés de formule (I) pour lesquels $R_3$ représente le groupement hydroxyle et $R_2$ et $R_4$ représentent chacun simultanément un groupement tert.butyl, leurs isomères et leurs sels d'addition à une base pharmaceutiquement acceptable.

**4.** Procédé selon la revendication 1 de préparation de composés de formule (I) pour lesquels Z forme avec T un chaînon $(CH_2)_n$-CH(E) ou $(CH_2)_n$-CH($CH_2$E') avec n entier égal à 0, 1, 2 ou 3 étant entendu que dans ce cas Z est porté par un carbone voisin du carbone porteur du groupement acylé, et E ou E' représentent un atome d'hydrogène, un groupement alkyle inférieur, un groupement phényle, hétéroaryle, ou phényle substitué ou hétéroaryle substitué,
leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable.

**5.** Procédé selon la revendication 1 de préparation de composés de formule (I) pour lesquels Z et T représentent simultanément un atome d'hydrogène, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

**6.** Procédé selon la revendication 1 de préparation de composés de formule (I) pour lesquel Y représente une liaison simple ou un groupement $CH_2$, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable

**7.** Procédé selon la revendication 1 de préparation de composés de formule (I) pour lesquels X représente un atome d'oxygène ou de soufre, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

**8.** Procédé selon la revendication 4 de préparation de composés de formule (I) pour lequel E représente un groupement phényle éventuellement substitué par un ou plusieurs groupements choisis parmi hydroxyle et alkyle inférieur, leurs isomères et leurs sels d'addition à une base pharmaceutiquement acceptable.

**9.** Procédé selon la revendication 1 de préparation du composé de formule (I) qui est la 7-(3',5' ditert.butyl 4'-hydroxy cinnamoyl) [1,4]benzothiazin-3-one, ses isomères et ses sels d'addition à une base pharmaceutiquement acceptable.

**10.** Procédé selon la revendication 1 de préparation du composé de formule (I) qui est la 6-(3',5'-ditert.butyl 4'-hydroxy cinnamoyl) benzoxazolinone, ses isomères et ses sels d'addition à une base pharmaceutiquement acceptable.

**11.** Procédé selon la revendication 1 de préparation du composé de formule (I) qui est la 2,3-dihydro 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

**12.** Procédé selon la revendication 1 de préparation du composé de formule (I) qui est la [4H] 2,3-dihydro 3,8-dioxo 2,2,4-triméthyl 6-(3',5'-ditert.butyl 4'-hydroxy phényl) 7-(3',5'-ditert.butyl 4'-hydroxybenzylidène) cyclopenta [g] 1,4-benzothiazine, ses isomères et ses sels d'addition à une base pharmaceutiquement acceptable.

**13.** Procédé selon la revendication 1 de préparation du composé de formule (I) qui est [4H] 2,3-dihydro 3,8-dioxo 4-méthyl 6-phényl 7-(3',5'-ditert.butyl 4'-hydroxy benzylidène) cyclopenta [g] 1,4-benzothiazine, ses isomères et ses sels d'addition à une base pharmaceutiquement acceptable.

**14.** Procédé selon la revendication 1 de préparation du composé de formule (I) qui est la 2,3-dihydro 3-méthyl 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

**15.** Procédé selon la revendication 1 de préparation du composé de formule (I) qui est la 2,3-dihydro 5-phényl 6-(3',5' ditert.butyl 4'-hydroxy benzylidène) cyclopenta [f] benzothiazol 2,7-dione, ses isomères et ses sels d'addition à une base pharmaceutiquement acceptable.

**16.** Procédé de préparation de composition pharmaceutique contenant comme principe actif au moins un composé obtenu selon la revendication 1 en combinaison avec un ou plusieurs excipients ou véhicules inertes non toxique pharmaceutiquement acceptable.

**17.** Procédé de préparation de composition pharmaceutique selon la revendication 16 contenant au moins un principe actif obtenu selon l'une des revendications 1 à 15 utilisables dans le traitement de l'inflammation, la prévention et le traitement des troubles plaquettaires dans les hypercholestérolémies, hypertriglycéridémies, dans le diabète non insulino dépendant ainsi que de ses complications.

**Revendications pour l'Etat contractant suivant : GR**

**1.** Procédé de préparation de composés de formule (I) ::

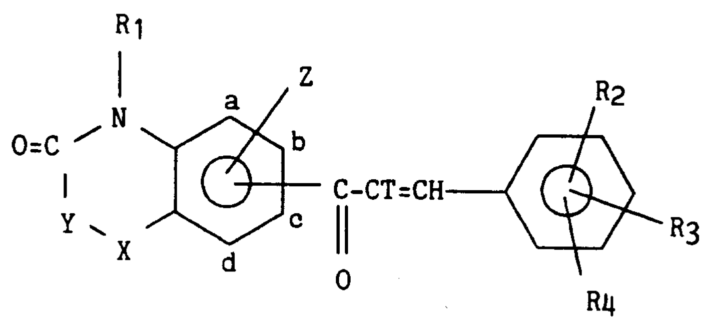

(I)

dans laquelle :

$R_1$ représente un atome d'hydrogène ou un radical alkyle inférieur,

X représente :

- . un atome d'oxygène ou de soufre,
- . un groupement $CH_2$ à la condition que dans ce cas Y représente un atome d'oxygène ou de soufre,

Y représente :

- . une liaison simple ou un groupement $CR_7R_8$ où $R_7$ et $R_8$ identiques ou différents représentent indépendamment l'un de l'autre un atome d'hydrogène, un groupement alkyle inférieur, un groupement phényle, un groupement phényle substitué, un groupement phényl alkyle ou un groupement phényl alkyle substitué,
- . un atome d'oxygène ou de soufre à la condition que dans ce cas X représente un groupement $CH_2$,

Z représente ou bien un atome d'hydrogène et dans ce cas T représente également un atome d'hydrogène ou bien Z forme avec T un chaînon $-(CH_2)_n-CH(E)-$ ou $(CH_2)_n-CH(CH_2E')$ avec n entier égal a 0, 1, 2 ou 3 étant entendu que dans ce cas Z est porté par un carbone voisin du carbone porteur du groupement acylé, et E ou E' représentent un atome d'hydrogène, un groupement alkyle inférieur, un groupement phényle, hétéroaryle, ou phényle substitué ou hétéroaryle substitué,

le terme substitué affectant les termes phényle et hétéroaryle et phényle alkyle dans la définition de $R_7$ et $R_8$ et E et E' signifiant que ces groupements peuvent être substitués par un ou plusieurs substituants choisis parmi alkyle inférieur, alkoxy inférieur, halogène, trifluorométhyle ou hydroxyle,

$R_2$, $R_3$ et $R_4$ identiques ou différents représentent indépendamment l'un de l'autre un groupement hydroxy ou alkoxy inférieur ou un groupement alkyle inférieur aux conditions que :

. lorsque X représente l'oxygène, Y représente une liaison simple et Z représente un atome d'hydrogène, l'un au moins de $R_2$, $R_3$ et $R_4$ est différent du groupement méthoxy,

. lorsque X représente l'oxygène, Y représente un groupement $CR_7R_8$ et Z représente un atome d'hydrogène, l'un au moins de $R_2$, $R_3$, $R_4$ n'est pas un groupement alkyle,

leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable,

étant entendu que par alkyle inférieur ou alkoxy inférieur, on entend des groupements linéaires ou ramifiés comprenant de 1 à 6 atomes de carbone,

que par hétéroaryle on entend des groupements non saturés mono ou bicycliques comprenant de 5 à 12 atomes autres que l'hydrogène et incluant dans un squelette carboné de 1 à 3 hétéroatomes choisis parmi azote, oxygène ou soufre,

caractérisé en ce que l'on fait réagir un composé de formule (II) :

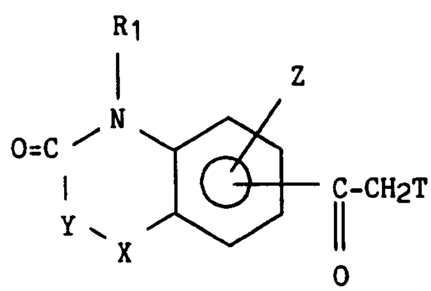

(II)

dans laquelle $R_1$, X, Y, Z et T ont la même signification que dans la formule (I),

en milieu organique acide avec un aldéhyde de formule (III) :

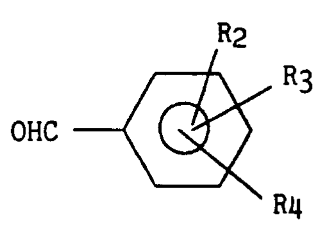

(III)

dans laquelle $R_2$, $R_3$ et $R_4$ ont la même définition que dans la formule (I),

pour conduire, après éventuelle neutralisation du milieu réactionnel, à un dérivé de formule (I), que l'on purifie si nécessaire par une technique choisie parmi chromatographie ou cristallisation, dont on sépare les isomères, le cas échéant et que l'on salifie, si on le désire, lorsque $R_1$ représente un atome d'hydrogène ou l'un au moins des groupements $R_2$, $R_3$ et $R_4$ représente un groupement hydroxyle, par une base pharmaceutiquement acceptable.

**2.** Procédé de préparation de composés de formule (II) selon la revendication 1 pour lesquels Z forme avec T un chaînon $-(CH_2)_n-CH(E)$ ou $(CH_2)_n-CH(CH_2E')$ à l'exception des dérivés pour lesquels X représente un atome d'oxygène, Y représente une liaison simple, E représente un groupement méthyle ou E' un atome d'hydrogène,
caractérisé par ce que l'on cyclise en milieu acide un dérivé de formule (IV) :

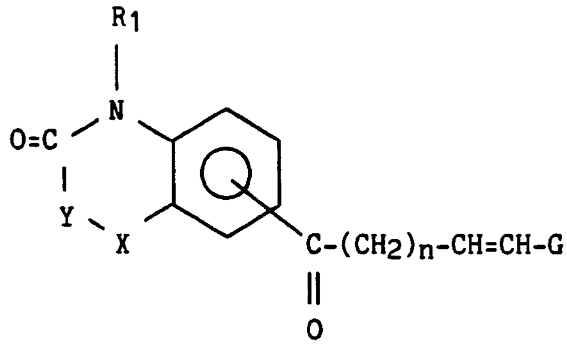

**(IV)**

avec n, Y, X et $R_1$ ayant la même définition que dans la formule (I) et G représente E ou $CH_2E'$,
dérivé de formule (IV) que l'on obtiendra,

- soit lorsque Y représente une liaison simple, par acylation en milieu acide d'un dérivé de formule (V) :

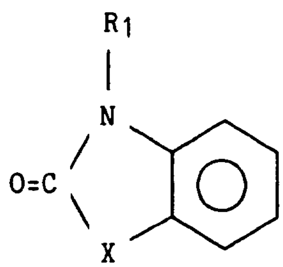

**(V)**

dans laquelle $R_1$ et X ont la même définition que dans la formule (I),
par un chlorure d'acide de formule (VI) :

$ClOC-(CH_2)_nCH=CH-E$ **(VI)**

dans laquelle n et E ont la même définition que dans la formule (I),
ou par un dérivé de formule (VII) :

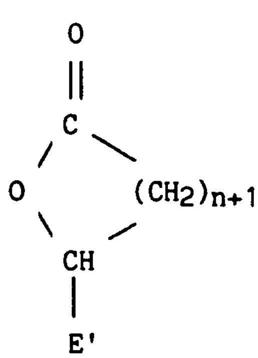

**(VII)**

dans laquelle n et E' ont la même définition que dans la formule (I),

- soit lorsque n = 0 par condensation d'un dérivé de formule (VIII) :

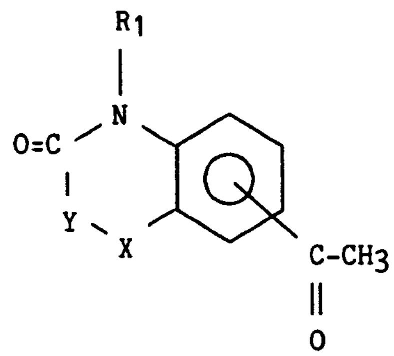

**(VIII)**

ou X, Y, $R_1$ et n ont la même définition que dans la formule (I), avec un aldéhyde de formule (IX) :

G-CHO **(IX)**

ou G a la même définition que précédemment,

- soit, lorsque G représente un atome d'hydrogène et n = 0, par déhydrohalogénation en milieu alcalin d'un dérivé de formule (X) :

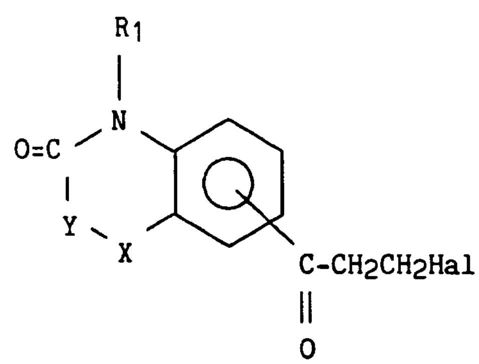

**(X)**

ou $R_1$, X, Y et n ont la même définition que précédemment et Hal représente un atome d'halogène,

avec le cas échéant purification et/ou séparation des isomères.

**3.** Procédé selon la revendication 1 de préparation de composés de formule (I) pour lesquels $R_3$ représente le groupement hydroxyle et $R_2$ et $R_4$ représentent chacun simultanément un groupement tert.butyl, leurs isomères et leurs sels d'addition à une base pharmaceutiquement acceptable.

**4.** Procédé selon la revendication 1 de préparation de composés de formule (I) pour lesquels Z forme avec T un chaînon $(CH_2)_n$-CH(E) ou $(CH_2)_n$-CH($CH_2$E') avec n entier égal à 0, 1, 2 ou 3 étant entendu que dans ce cas Z est porté par un carbone voisin du carbone porteur du groupement acylé, et E ou E' représentent un atome d'hydrogène, un groupement alkyle inférieur, un groupement phényle, hétéroaryle, ou phényle substitué ou hétéroaryle substitué,
leurs énantiomères, épimères, diastéréoisomères ainsi que lorsque $R_1$ représente un atome d'hydrogène ou lorsque l'un au moins des groupements $R_2$, $R_3$, $R_4$ représente un groupement hydroxyle, leurs sels d'addition à une base pharmaceutiquement acceptable.

**5.** Procédé selon la revendication 1 de préparation de composés de formule (I) pour lesquels Z et T représentent simultanément un atome d'hydrogène, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

6. Procédé selon la revendication 1 de préparation de composés de formule (I) pour lesquel Y représente une liaison simple ou un groupement $CH_2$, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

7. Procédé selon la revendication 1 de préparation de composés de formule (I) pour lesquels X représente un atome d'oxygène ou de soufre, leurs isomères ainsi que lorsque $R_1$ représente un atome d'hydrogène et l'un au moins des groupements $R_2$, $R_3$, $R_4$ représente un groupement hydroxyle, leurs sels d'addition à une base pharmaceutiquement acceptable.

8. Procédé selon la revendication 4 de préparation de composés de formule (I) pour lequel E représente un groupement phényle éventuellement substitué par un ou plusieurs groupements choisis parmi hydroxyle et alkyle inférieur, leurs isomères et leurs sels d'addition à une base pharmaceutiquement acceptable.

9. Procédé selon la revendication 1 de préparation du composé de formule (I) qui est la 7-(3',5' ditert.butyl 4'-hydroxy cinnamoyl) [l,4]benzothiazin-3-one, ses isomères et ses sels d'addition à une base pharmaceutiquement acceptable.

10. Procédé selon la revendication 1 de préparation du composé de formule (I) qui est la 6-(3',5'-ditert.butyl 4'-hydroxy cinnamoyl) benzoxazolinone, ses isomères et ses sels d'addition à une base pharmaceutiquement acceptable.

11. Procédé selon la revendication 1 de préparation du composé de formule (I) qui est la 2,3-dihydro 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

12. Procédé selon la revendication 1 de préparation du composé de formule (I) qui est la [4H] 2,3-dihydro 3,8-dioxo 2,2,4-triméthyl 6-(3',5'-ditert.butyl 4'-hydroxy phényl) 7-(3',5'-ditert.butyl 4'-hydroxybenzylidène) cyclopenta [g] 1,4-benzothiazine, ses isomères et ses sels d'addition à une base pharmaceutiquement acceptable.

13. Procédé selon la revendication 1 de préparation du composé de formule (I) qui est [4H] 2,3-dihydro 3,8-dioxo 4-méthyl 6-phényl 7-(3',5'-ditert.butyl 4'-hydroxy benzylidène) cyclopenta [g] 1,4-benzothiazine, ses isomères et ses sels d'addition à une base pharmaceutiquement acceptable.

14. Procédé selon la revendication 1 de préparation du composé de formule (I) qui est la 2,3-dihydro 3-méthyl 5-phényl 6-(3',5'-ditert.butyl 4'-hydroxy benzylidène) cyclopenta [f] benzoxazol 2,7-dione, ses isomères et ses sels d'addition à une base pharmaceutiquement acceptable.

15. Procédé selon la revendication 1 de préparation du composé de formule (I) qui est la 2,3-dihydro 5-phényl 6-(3',5' ditert.butyl 4'-hydroxy benzylidène) cyclopenta [f] benzothiazol 2,7-dione, ses isomères et ses sels d'addition à une base pharmaceutiquement acceptable.

16. Procédé de préparation de composition pharmaceutique contenant comme principe actif au moins un composé obtenu selon la revendication 1 en combinaison avec un ou plusieurs excipients ou véhicules inertes non toxique pharmaceutiquement acceptable.

17. Procédé de préparation de composition pharmaceutique selon la revendication 16 contenant au moins un principe actif obtenu selon l'une des revendications 1 à 15 utilisables dans le traitement de l'inflammation, la prévention et le traitement des troubles plaquettaires dans les hypercholestérolémies, hypertriglycéridémies, dans le diabète non insulino dépendant ainsi que de ses complications.

**Claims**

**Claims for the following Contracting States : AT, BE, CH, DE, DK, FR, GB, IT, LI, LU, NL, SE**

1. Compounds of formula (I):

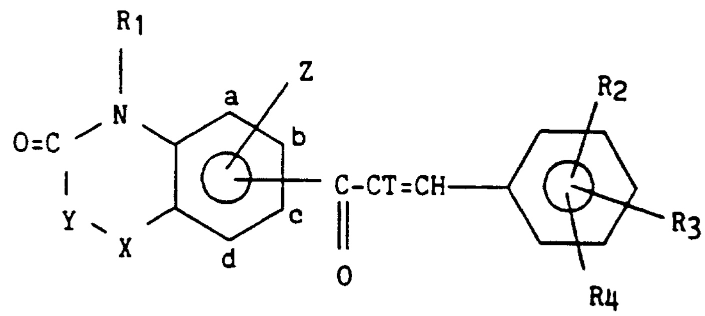

(I)

wherein:

$R_1$ represents a hydrogen atom or a lower alkyl radical,

X represents:

. an oxygen or sulphur atom,

. a $CH_2$ group with the proviso that in that case Y represents an oxygen or sulphur atom,

Y represents:

. a single bond or a $CR_7R_8$ group wherein $R_7$ and $R_8$ are the same or different and each represents, independently of the other, a hydrogen atom, a lower alkyl group, a phenyl group, a substituted phenyl group, a phenylalkyl group or a substituted phenylalkyl group,

. an oxygen or sulphur atom with the proviso that in that case X represents a $CH_2$ group,

Z represents a hydrogen atom, in which case T also represents a hydrogen atom, or Z forms together with T a $-(CH_2)_n-CH(E)-$ or $(CH_2)_n-CH(CH_2E')$ group in which n is an integer 0, 1, 2 or 3, it being understood that in that case Z is carried by a carbon atom vicinal to the carbon atom carrying the acyl group, and E or E' represents a hydrogen atom, a lower alkyl group, or a phenyl, heteroaryl, substituted phenyl or substituted heteroaryl group,

the term "substituted" governing the terms phenyl and heteroaryl and phenylalkyl in the definition of $R_7$ and $R_8$ and E and E' indicating that those groups may be substituted by one or more substituents selected from lower alkyl, lower alkoxy, halogen, trifluoromethyl and hydroxy,

$R_2$, $R_3$ and $R_4$ are the same or different and each represents, independently of the others, a hydroxy or lower alkoxy group or a lower alkyl group with the provisos that :

. when X represents oxygen, Y represents a single bond and Z represents a hydrogen atom, at least one of $R_2$, $R_3$ and $R_4$ is other than a methoxy group,

. when X represents oxygen, Y represents a $CR_7R_8$ group and Z represents a hydrogen atom, at least one of $R_2$, $R_3$ and $R_4$ is not an alkyl group,

their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base,

there being understood by lower alkyl or lower alkoxy straight-chain or branched groups containing from 1 to 6 carbon atoms,

and there being understood by heteroaryl unsaturated mono- or bi-cyclic groups containing from 5 to 12 atoms other than hydrogen and including in a carbon skeleton from 1 to 3 hetero atoms selected from nitrogen, oxygen and sulphur.

2. Compounds according to claim 1 wherein $R_3$ represents a hydroxy group and $R_2$ and $R_4$ each represents, simultaneously, a tert-butyl group, their isomers and their addition salts with a pharmaceutically acceptable base.

3. Compounds according to claim 1 wherein Z forms together with T a $(CH_2)_n-CH(E)$ or $(CH_2)_n-CH(CH_2E')$ group in which n is an integer 0, 1, 2 or 3 it being understood that in that case Z is carried by a carbon

atom vicinal to the carbon atom carrying the acyl group, and E or E' represents a hydrogen atom, a lower alkyl group, or a phenyl, heteroaryl, substituted phenyl or substituted heteroaryl group, their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base.

**4.** Compounds according to claim 1 wherein Z and T simultaneously represent a hydrogen atom, their isomers and also, when $R_1$ represents a hydrogen atom and at least one of the groups $R_2$, $R_3$ and $R_4$ represents a hydroxy group, their addition salts with a pharmaceutically acceptable base.

**5.** Compounds according to claim 1 wherein Y represents a single bond or a $CH_2$ group, their isomers and also, when $R_1$ represents a hydrogen atom and at least one of the groups $R_2$, $R_3$ and $R_4$ represents a hydroxy group, their addition salts with a pharmaceutically acceptable base.

**6.** Compounds according to claim 1 wherein X represents an oxygen or sulphur atom, their isomers and also, when $R_1$ represents a hydrogen atom and at least one of the groups $R_2$, $R_3$ and $R_4$ represents a hydroxy group, their addition salts with a pharmaceutically acceptable base.

**7.** Compounds according to claim 3 wherein E represents a phenyl group optionally substituted by one or more groups selected from hydroxy and lower alkyl, their isomers and their addition salts with a pharmaceutically acceptable base.

**8.** Compound according to claim 1, which is 7-(3',5'-di-tert-butyl-4'-hydroxycinnamoyl)[1,4]benzothiazin-3-one, its isomers and its addition salts with a pharmaceutically acceptable base.

**9.** Compound according to claim 1, which is 6-(3',5'-di-tert-butyl-4'-hydroxycinnamoyl)benzoxazolinone, its isomers and its addition salts with a pharmaceutically acceptable base.

**10.** Compound according to claim 1, which is 2,3-dihydro-5-phenyl-6-(3',5'-di-tert-butyl-4'-hydroxybenzylidene)-cyclopenta[f]benzoxazole-2,7-dione, its isomers and its addition salts with a pharmaceutically acceptable base.

**11.** Compound according to claim 1, which is [4H]-2,3-dihydro-3,8-dioxo-2,2,4-trimethyl-6-(3',5'-di-tert-butyl-4'-hydroxyphenyl)-7-(3',5'-di-tert-butyl-4'-hydroxybenzylidene)cyclopenta[g]1,4-benzothiazine, its isomers and its addition salts with a pharmaceutically acceptable base.

**12.** Compound according to claim 1, which is [4H]-2,3-dihydro-3,8-dioxo-4-methyl-6-phenyl-7-(3',5'-di-tert-butyl-4'-hydroxybenzylidene)cyclopenta[g]1,4-benzothiazine, its isomers and its addition salts with a pharmaceutically acceptable base.

**13.** Compound according to claim 1, which is 2,3-dihydro-3-methyl-5-phenyl-6-(3',5'-di-tert-butyl-4'-hydroxybenzylidene)cyclopenta[f]benzoxazole-2,7-dione, its isomers and its addition salts with a pharmaceutically acceptable base.

**14.** Compound according to claim 1, which is 2,3-dihydro-5-phenyl-6-(3',5'-di-tert-butyl-4'-hydroxybenzylidene)-cyclopenta[f]benzothiazole-2,7-dione, its isomers and its addition salts with a pharmaceutically acceptable base.

**15.** Process for the preparation of compounds of formula (I) according to claim 1, characterised in that a compound of formula (II):

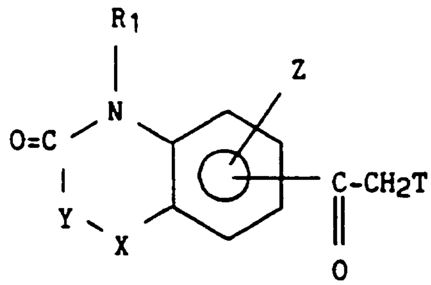

(II)

wherein $R_1$, X, Y, Z and T are as defined for formula (I), is reacted in an organic acid medium with an aldehyde of formula (III) :

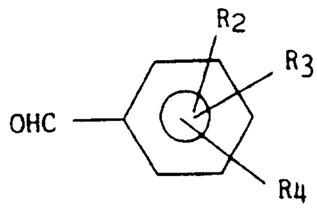

(III)

wherein $R_2$, $R_3$ and $R_4$ are as defined for formula (I),
to yield, after optional neutralisation of the reaction mixture, a compound of formula (I), which is purified, if necessary, by a technique selected from chromatography and crystallisation, is separated into its isomers, if required, and is, if desired, converted into a salt when $R_1$ represents a hydrogen atom or at least one of the groups $R_2$, $R_3$ and $R_4$ represents a hydroxy group, with a pharmaceutically acceptable base.

**16.** Compounds of formula (II)

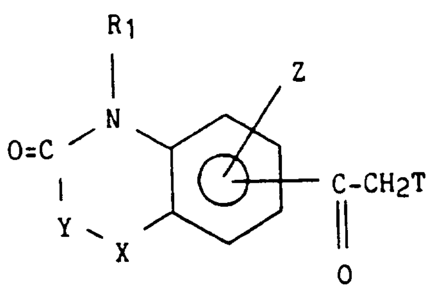

(II)

wherein:

$R_1$ represents a hydrogen atom or a lower alkyl radical,

X represents:
- . an oxygen or sulphur atom,
- . a $CH_2$ group with the proviso that in that case Y represents an oxygen or sulphur atom,

Y represents:
- . a single bond or a $CR_7R_8$ group wherein $R_7$ and $R_8$ are the same or different and each represents, independently of the other, a hydrogen atom, a lower alkyl group, a phenyl group, a substituted phenyl group, a phenylalkyl group or a substituted phenylalkyl group,
- . an oxygen or sulphur atom with the proviso that in that case X represents a $CH_2$ group,

Z forms together with T a $-(CH_2)_n-CH(E)-$ or $(CH_2)_n-CH(CH_2E')$ group in which n is an integer 0, 1, 2 or 3, it being understood that in that case Z is carried by a carbon atom vicinal to the carbon atom carrying the acyl group, and E or E' represents a hydrogen atom, a lower alkyl group, or a phenyl, heteroaryl, substituted phenyl or substituted heteroaryl group, with the

exception of compounds in which X represents an oxygen atom, Y represents a single bond and E represents a methyl group or E' represents a hydrogen atom, and also, when $R_1$ represents a hydrogen atom or at least one of the groups $R_2$, $R_3$ and $R_4$ represents a hydroxy group, their addition salts with a base, for use as intermediates in the synthesis of compounds of formula (I),

the term "substituted" governing the terms phenyl and heteroaryl and phenylalkyl in the definition of $R_7$ and $R_8$ and E and E' indicating that those groups may be substituted by one or more substituents selected from lower alkyl, lower alkoxy, halogen, trifluoromethyl and hydroxy.

**17.** Pharmaceutical composition comprising as active ingredient at least one compound according to claim 1 in combination with one or more pharmaceutically acceptable inert non-toxic excipients or carriers.

**18.** Pharmaceutical composition according to claim 17 comprising at least one active ingredient according to any one of claims 1 to 15 that can be used in the treatment of inflammation, atherosclerosis, the prevention and treatment of platelet disorders in hypercholesterolaemia, hypertriglyceridaemia, in non-insulin-dependent diabetes and also complications thereof.

**Claims for the following Contracting State : ES**

**1.** Process for the preparation of compounds of formula (I):

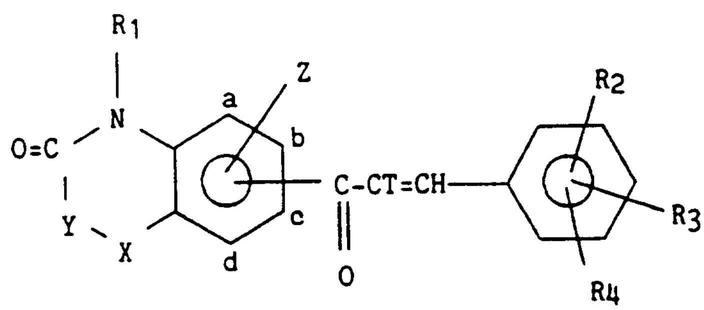

(I)

wherein:

$R_1$ represents a hydrogen atom or a lower alkyl radical,

X represents:

. an oxygen or sulphur atom,

. a $CH_2$ group with the proviso that in that case Y represents an oxygen or sulphur atom,

Y represents:

. a single bond or a $CR_7R_8$ group wherein $R_7$ and $R_8$ are the same or different and each represents, independently of the other, a hydrogen atom, a lower alkyl group, a phenyl group, a substituted phenyl group, a phenylalkyl group or a substituted phenylalkyl group,

. an oxygen or sulphur atom with the proviso that in that case X represents a $CH_2$ group,

Z represents a hydrogen atom, in which case T also represents a hydrogen atom, or Z forms together with T a $-(CH_2)_n-CH(E)-$ or $(CH_2)_n-CH(CH_2E')$ group in which n is an integer 0, 1, 2 or 3, it being understood that in that case Z is carried by a carbon atom vicinal to the carbon atom carrying the acyl group, and E or E' represents a hydrogen atom, a lower alkyl group, or a phenyl, heteroaryl, substituted phenyl or substituted heteroaryl group,

the term "substituted" governing the terms phenyl and heteroaryl and phenylalkyl in the definition of $R_7$ and $R_8$ and E and E' indicating that those groups may be substituted by one or more substituents selected from lower alkyl, lower alkoxy, halogen, trifluoromethyl and hydroxy,

$R_2$, $R_3$ and $R_4$ are the same or different and each represents, independently of the others, a hydroxy or lower alkoxy group or a lower alkyl group with the provisos that :

. when X represents oxygen, Y represents a single bond and Z represents a hydrogen atom, at least one of $R_2$, $R_3$ and $R_4$ is other than a methoxy group,

. when X represents oxygen, Y represents a $CR_7R_8$ group and Z represents a hydrogen atom, at least one of $R_2$, $R_3$ and $R_4$ is not an alkyl group,

their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base,

there being understood by lower alkyl or lower alkoxy straight-chain or branched groups containing from 1 to 6 carbon atoms,

and there being understood by heteroaryl unsaturated mono- or bi-cyclic groups containing from 5 to 12 atoms other than hydrogen and including in a carbon skeleton from 1 to 3 hetero atoms selected from nitrogen, oxygen and sulphur,

characterised in that a compound of formula (II):

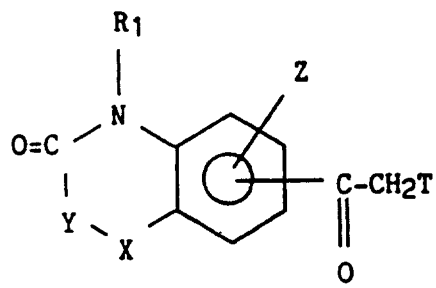

(II)

wherein $R_1$, X, Y, Z and T are as defined for formula (I), is reacted in an organic acid medium with an aldehyde of formula (III) :

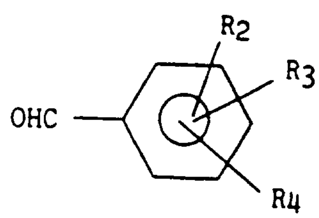

(III)

wherein $R_2$, $R_3$ and $R_4$ are as defined for formula (I),

to yield, after optional neutralisation of the reaction mixture, a compound of formula (I), which is purified, if necessary, by a technique selected from chromatography and crystallisation, is separated into its isomers, if required, and is, if desired, converted into a salt when $R_1$ represents a hydrogen atom or at least one of the groups $R_2$, $R_3$ and $R_4$ represents a hydroxy group, with a pharmaceutically acceptable base.

**2.** Process for the preparation of compounds of formula (II) according to claim 1 wherein Z forms together with T a $-(CH_2)_n-CH(E)-$ or $(CH_2)_n-CH(CH_2E')$ group with the exception of compounds wherein X represents an oxygen atom, Y represents a single bond and E represents a methyl group or E' represents a hydrogen atom, characterised in that a compound of formula (IV) :

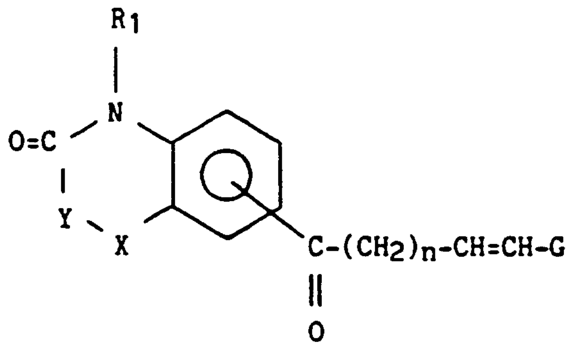

(IV)

wherein n, Y, X and $R_1$ are as defined for formula (I) and G represents E or $CH_2E'$, is cyclised in acid medium,
which compound of formula (IV) will be obtained

- when Y represents a single bond, by acylation in acid medium of a compound of formula (V) :

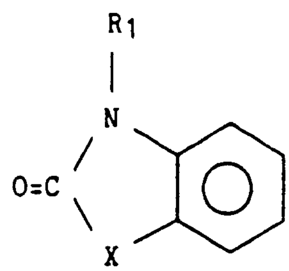

(V)

wherein $R_1$ and X are as defined for formula (I),
by an acid chloride of formula (VI) :

$ClOC\text{-}(CH_2)_nCH=CH\text{-}E$ (VI)

wherein n and E are as defined for formula (I),
or by a compound of formula (VII) :

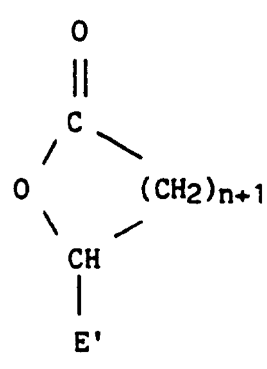

(VII)

wherein n and E' are as defined for formula (I),

- when n = O, by condensation of a compound of formula (VIII) :

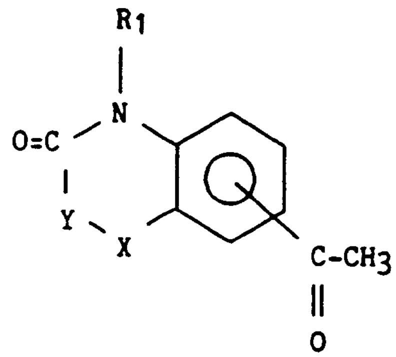

(VIII),

wherein X, Y, $R_1$ and n are as defined for formula (I), with an aldehyde of formula (IX) :

G-CHO (IX)

wherein G is as defined hereinbefore,
- or when G represents a hydrogen atom and n = 0, by dehydrohalogenation in alkaline medium of a compound of formula (X) :

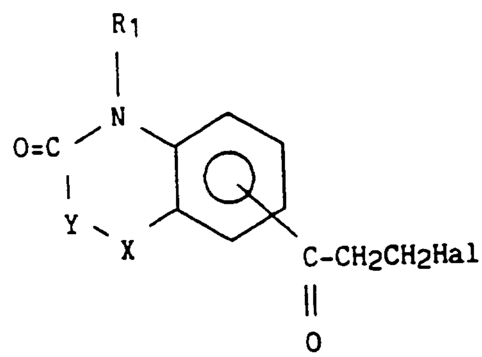

(X)

wherein $R_1$, X, Y and n are as defined hereinbefore and Hal represents a halogen atom,
if required with purification and/or separation of the isomers.

**3.** Process according to claim 1 for the preparation of compounds of formula (I) wherein $R_3$ represents a hydroxy group and $R_2$ and $R_4$ each represents, simultaneously, a tert-butyl group, their isomers and their addition salts with a pharmaceutically acceptable base.

**4.** Process according to claim 1 for the preparation of compounds of formula (I) wherein Z forms together with T a $(CH_2)_n$-CH(E) or $(CH_2)_n$-CH($CH_2$E') group in which n is an integer 0, 1, 2 or 3 it being understood that in that case Z is carried by a carbon atom vicinal to the carbon atom carrying the acyl group, and E or E' represents a hydrogen atom, a lower alkyl group or a phenyl, heteroaryl, substituted phenyl or substituted heteroaryl group,
their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base.

**5.** Process according to claim 1 for the preparation of compounds of formula (I) wherein Z and T simultaneously represent a hydrogen atom, their isomers and also, when $R_1$ represents a hydrogen atom and at least one of the groups $R_2$, $R_3$ and $R_4$ represents a hydroxy group, their addition salts with a pharmaceutically acceptable base.

**6.** Process according to claim 1 for the preparation of compounds of formula (I) wherein Y represents a single bond or a $CH_2$ group, their isomers and also, when $R_1$ represents a hydrogen atom and at least one of the groups $R_2$, $R_3$ and $R_4$ represents a hydroxy group, their addition salts with a pharmaceutically acceptable base.

**7.** Process according to claim 1 for the preparation of compounds of formula (I) wherein X represents an oxygen or sulphur atom, their isomers and also, when $R_1$ represents a hydrogen atom and at least one of the groups $R_2$, $R_3$ and $R_4$ represents a hydroxy group, their addition salts with a pharmaceutically acceptable base.

**8.** Process according to claim 4 for the preparation of compounds of formula (I) wherein E represents a phenyl group optionally substituted by one or more groups selected from hydroxy and lower alkyl, their isomers and their addition salts with a pharmaceutically acceptable base.

**9.** Process according to claim 1 for the preparation of the compound of formula (I) which is 7-(3',5'-di-tert-butyl-4'-hydroxycinnamoyl)[1,4]benzothiazin-3-one, its isomers and its addition salts with a pharmaceutically acceptable base.

**10.** Process according to claim 1 for the preparation of the compound of formula (I) which is 6-(3',5'-di-tert-butyl-4'-hydroxycinnamoyl)benzoxazolinone, its isomers and its addition salts with a pharmaceutically acceptable base.

**11.** Process according to claim 1 for the preparation of the compound of formula (I) which is 2,3-dihydro-5-phenyl-6-(3',5'-di-tert-butyl-4'-hydroxybenzylidene)-cyclopenta[f]benzoxazole-2,7-dione, its isomers and its addition salts with a pharmaceutically acceptable base.

**12.** Process according to claim 1 for the preparation of the compound of formula (I) which is [4H]-2,3-dihydro-3,8-dioxo-2,2,4-trimethyl-6-(3',5'-di-tert-butyl-4'-hydroxyphenyl)-7-(3',5'-di-tert-butyl-4'-hydroxybenzylidene)cyclopenta[g]1,4-benzothiazine, its isomers and its addition salts with a pharmaceutically acceptable base.

**13.** Process according to claim 1 for the preparation of the compound of formula (I) which is [4H]-2,3-dihydro-3,8-dioxo-4-methyl-6-phenyl-7-(3',5'-di-tert-butyl-4'-hydroxybenzylidene)cyclopenta[g]1,4-benzothiazine, its isomers and its addition salts with a pharmaceutically acceptable base.

**14.** Process according to claim 1 for the preparation of the compound of formula (I) which is 2,3-dihydro-3-methyl-5-phenyl-6-(3',5'-di-tert-butyl-4'-hydroxybenzylidene)cyclopenta[f]benzoxazole-2,7-dione, its isomers and its addition salts with a pharmaceutically acceptable base.

**15.** Process according to claim 1 for the preparation of the compound of formula (I) which is 2,3-dihydro-5-phenyl-6-(3',5'-di-tert-butyl-4'-hydroxybenzylidene)-cyclopenta[f]benzothiazole-2,7-dione, its isomers and its addition salts with a pharmaceutically acceptable base.

**16.** Process for the preparation of a pharmaceutical composition comprising as active ingredient at least one compound obtained in accordance with claim 1 in combination with one or more pharmaceutically acceptable inert non-toxic excipients or carriers.

**17.** Process for the preparation of a pharmaceutical composition according to claim 16 comprising at least one active ingredient obtained in accordance with any one of claims 1 to 15 that can be used in the treatment of inflammation, the prevention and treatment of platelet disorders in hypercholesterolaemia, hypertriglyceridaemia, in non-insulin-dependent diabetes and also complications thereof.

**Claims for the following Contracting State : GR**

**1.** Process for the preparation of compounds of formula (I):

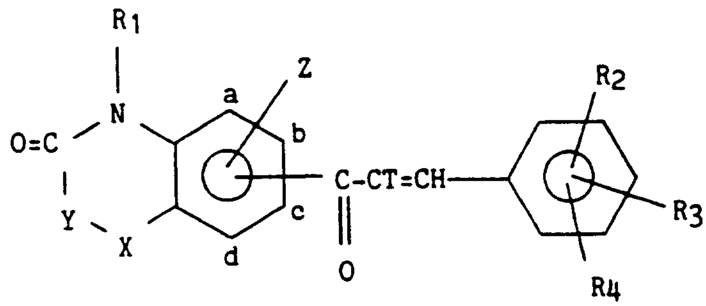

(I)

wherein:

$R_1$ represents a hydrogen atom or a lower alkyl radical,

X represents:
- . an oxygen or sulphur atom,
- . a $CH_2$ group with the proviso that in that case Y represents an oxygen or sulphur atom,

Y represents:
- . a single bond or a $CR_7R_8$ group wherein $R_7$ and $R_8$ are the same or different and each represents, independently of the other, a hydrogen atom, a lower alkyl group, a phenyl group, a substituted phenyl group, a phenylalkyl group or a substituted phenylalkyl group,
- . an oxygen or sulphur atom with the proviso that in that case X represents a $CH_2$ group,

Z represents a hydrogen atom, in which case T also represents a hydrogen atom, or Z forms together with T a $-(CH_2)_n-CH(E)-$ or $(CH_2)_n-CH(CH_2E')$ group in which n is an integer 0, 1, 2 or 3, it being understood that in that case Z is carried by a carbon atom vicinal to the carbon atom carrying the acyl group, and E or E' represents a hydrogen atom, a lower alkyl group, or a phenyl, heteroaryl, substituted phenyl or substituted heteroaryl group,

the term "substituted" governing the terms phenyl and heteroaryl and phenylalkyl in the definition of $R_7$ and $R_8$ and E and E' indicating that those groups may be substituted by one or more substituents selected from lower alkyl, lower alkoxy, halogen, trifluoromethyl and hydroxy,

$R_2$, $R_3$ and $R_4$ are the same or different and each represents, independently of the others, a hydroxy or lower alkoxy group or a lower alkyl group with the provisos that :
- . when X represents oxygen, Y represents a single bond and Z represents a hydrogen atom, at least one of $R_2$, $R_3$ and $R_4$ is other than a methoxy group,
- . when X represents oxygen, Y represents a $CR_7R_8$ group and Z represents a hydrogen atom, at least one of $R_2$, $R_3$ and $R_4$ is not an alkyl group,

their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base,

there being understood by lower alkyl or lower alkoxy straight-chain or branched groups containing from 1 to 6 carbon atoms,

and there being understood by heteroaryl unsaturated mono- or bi-cyclic groups containing from 5 to 12 atoms other than hydrogen and including in a carbon skeleton from 1 to 3 hetero atoms selected from nitrogen, oxygen and sulphur,

characterised in that a compound of formula (II):

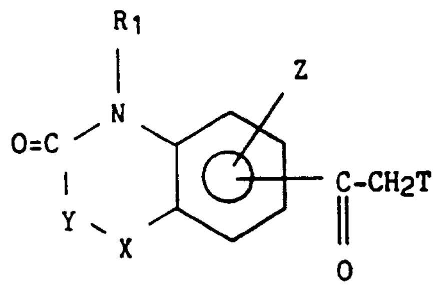

(II)

wherein $R_1$, X, Y, Z and T are as defined for formula (I), is reacted in an organic acid medium with an aldehyde of formula (III) :

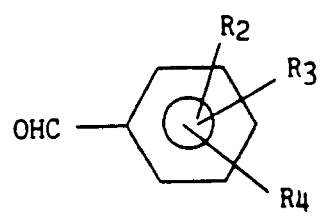

(III)

wherein $R_2$, $R_3$ and $R_4$ are as defined for formula (I),
to yield, after optional neutralisation of the reaction mixture, a compound of formula (I), which is purified, if necessary, by a technique selected from chromatography and crystallisation, is separated into its isomers, if required, and is, if desired, converted into a salt when $R_1$ represents a hydrogen atom or at least one of the groups $R_2$, $R_3$ and $R_4$ represents a hydroxy group, with a pharmaceutically acceptable base.

**2.** Process for the preparation of compounds of formula (II) according to claim 1 wherein Z forms together with T a $-(CH_2)_n-CH(E)-$ or $(CH_2)_n-CH(CH_2E')$ group with the exception of compounds wherein X represents an oxygen atom, Y represents a single bond and E represents a methyl group or E' represents a hydrogen atom, characterized in that a compound of formula (IV) :

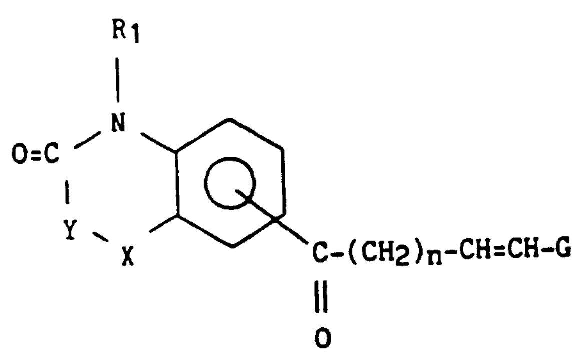

(IV)

wherein n, Y, X and $R_1$ are as defined for formula (I) and G represents E or $CH_2E'$, is cyclised in acid medium,
which compound of formula (IV) will be obtained

- when Y represents a single bond, by acylation in acid medium of a compound of formula (V) :

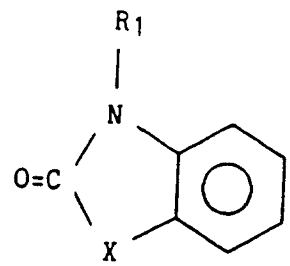

(V)

wherein $R_1$ and X are as defined for formula (I), by an acid chloride of formula (VI) :

$ClOC\text{-}(CH_2)_nCH = CH\text{-}E$ (VI)

wherein n and E are as defined for formula (I), or by a compound of formula (VII) :

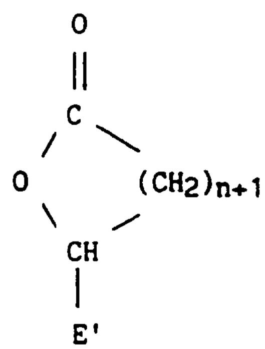

(VII)

wherein n and E' are as defined for formula (I),
- when n = O, by condensation of a compound of formula (VIII) :

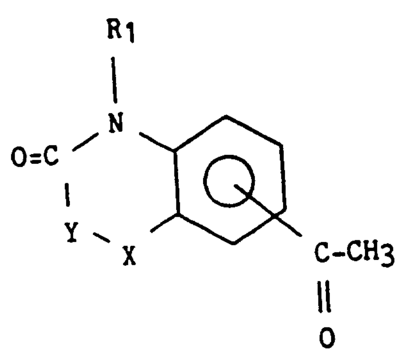

(VIII),

wherein X, Y, $R_1$ and n are as defined for formula (I), with an aldehyde of formula (IX) :;

G-CHO (IX)

wherein G is as defined hereinbefore,
- or when G represents a hydrogen atom and n = 0, by dehydrohalogenation in alkaline medium of a compound of formula (X) :

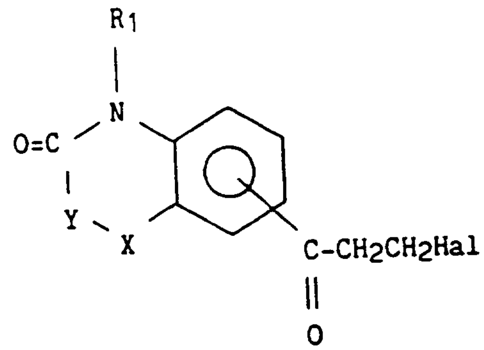

(X)

wherein $R_1$, X, Y and n are as defined hereinbefore and Hal represents a halogen atom, if required with purification and/or separation of the isomers.

**3.** Process according to claim 1 for the preparation of compounds of formula (I) wherein $R_3$ represents a hydroxy group and $R_2$ and $R_4$ each represents, simultaneously, a tert-butyl group, their isomers and their addition salts with a pharmaceutically acceptable base.

**4.** Process according to claim 1 for the preparation of compounds of formula (I) wherein Z forms together with T a $(CH_2)_n$-CH(E) or $(CH_2)_n$-$CH(CH_2E')$ group in which n is an integer 0, 1, 2 or 3 it being understood that in that case Z is carried by a carbon atom vicinal to the carbon atom carrying the acyl group, and E or E' represents a hydrogen atom, a lower alkyl group or a phenyl, heteroaryl, substituted phenyl or substituted heteroaryl group,
their enantiomers, epimers, diasteroisomers and also, when $R_1$ represents a hydrogen atom or when at least one of the groups $R_2$, $R_3$ and $R_4$ represents a hydroxy group, their addition salts with a pharmaceutically acceptable base.

**5.** Process according to claim 1 for the preparation of compounds of formula (I) wherein Z and T simultaneously represent a hydrogen atom, their isomers and also, when $R_1$ represents a hydrogen atom and at least one of the groups $R_2$, $R_3$ and $R_4$ represents a hydroxy group, their addition salts with a pharmaceutically acceptable base.

**6.** Process according to claim 1 for the preparation of compounds of formula (I) wherein Y represents a single bond or a $CH_2$ group, their isomers and also, when $R_1$ represents a hydrogen atom and at least one of the groups $R_2$, $R_3$ and $R_4$ represents a hydroxy group, their addition salts with a pharmaceutically acceptable base.

**7.** Process according to claim 1 for the preparation of compounds of formula (I) wherein X represents an oxygen or sulphur atom, their isomers and also, when $R_1$ represents a hydrogen atom and at least one of the groups $R_2$, $R_3$ and $R_4$ represents a hydroxy group, their addition salts with a pharmaceutically acceptable base.

**8.** Process according to claim 4 for the preparation of compounds of formula (I) wherein E represents a phenyl group optionally substituted by one or more groups selected from hydroxy and lower alkyl, their isomers and their addition salts with a pharmaceutically acceptable base.

**9.** Process according to claim 1 for the preparation of the compound of formula (I) which is 7-(3',5'-di-tert-butyl-4'-hydroxycinnamoyl)[1,4]benzothiazin-3-one, its isomers and its addition salts with a pharmaceutically acceptable base.

**10.** Process according to claim 1 for the preparation of the compound of formula (I) which is 6-(3',5'-di-tert-butyl-4'-hydroxycinnamoyl)benzoxazolinone, its isomers and its addition salts with a pharmaceutically acceptable base.

11. Process according to claim 1 for the preparation of the compound of formula (I) which is 2,3-dihydro-5-phenyl-6-(3',5'-di-tert-butyl-4'-hydroxybenzylidene)-cyclopenta[f]benzoxazole-2,7-dione, its isomers and its addition salts with a pharmaceutically acceptable base.

12. Process according to claim 1 for the preparation of the compound of formula (I) which is [4H]-2,3-dihydro-3,8-dioxo-2,2,4-trimethyl-6-(3',5'-di-tert-butyl-4'-hydroxyphenyl)-7-(3',5'-di-tert-butyl-4'-hydroxybenzylidene)cyclopenta[g]1,4-benzothiazine, its isomers and its addition salts with a pharmaceutically acceptable base.

13. Process according to claim 1 for the preparation of the compound of formula (I) which is [4H]-2,3-dihydro-3,8-dioxo-4-methyl-6-phenyl-7-(3',5'-di-tert-butyl-4'-hydroxybenzylidene)cyclopenta[g]1,4-benzothiazine, its isomers and its addition salts with a pharmaceutically acceptable base.

14. Process according to claim 1 for the preparation of the compound of formula (I) which is 2,3-dihydro-3-methyl-5-phenyl-6-(3',5'-di-tert-butyl-4'-hydroxybenzylidene)cyclopenta[f]benzoxazole-2,7-dione, its isomers and its addition salts with a pharmaceutically acceptable base.

15. Process according to claim 1 for the preparation of the compound of formula (I) which is 2,3-dihydro-5-phenyl-6-(3',5'-di-tert-butyl-4'-hydroxybenzylidene)-cyclopenta[f]benzothiazole-2,7-dione, its isomers and its addition salts with a pharmaceutically acceptable base.

16. Process for the preparation of a pharmaceutical composition comprising as active ingredient at least one compound obtained in accordance with claim 1 in combination with one or more pharmaceutically acceptable inert non-toxic excipients or carriers.

17. Process for the preparation of a pharmaceutical composition according to claim 16 comprising at least one active ingredient obtained in accordance with any one of claims 1 to 15 that can be used in the treatment of inflammation, the prevention and treatment of platelet disorders in hypercholesterolaemia, hypertriglyceridaemia, in non-insulin-dependent diabetes and also complications thereof.

**Patentansprüche**
**Patentansprüche für folgende Vertragsstaaten : AT, BE, CH, DE, DK, FR, GB, IT, LI, LU, NL, SE**

1. Verbindungen der Formel (I):

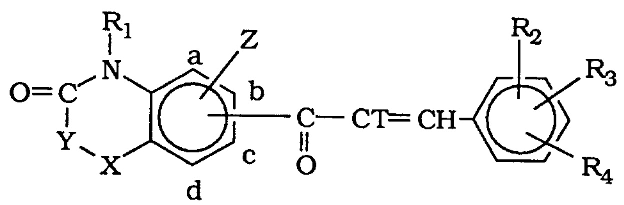

(I)

in der:

$R_1$ ein Wasserstoffatom oder eine Niedrigalkylgruppe,

X

. ein Sauerstoff- oder Schwefelatom,
. eine $CH_2$-Gruppe mit der Maßgabe, daß in diesem Fall Y ein Sauerstoff-oder Schwefelatom darstellt.

Y

. eine Einfachbindung oder eine Gruppe $CR_7R_8$, in der $R_7$ und $R_8$, die gleichartig oder verschieden sein können, unabhängig voneinander Wasserstoffatome, Niedrigalkylgruppen, Phenylgruppen, substituierte Phenylgruppen, Phenylalkylgruppen oder substituierte Phenylalkylgruppen darstellen,
. ein Sauerstoff- oder Schwefelatom mit der Maßgabe, daß in diesem Fall X eine $CH_2$-Gruppe darstellt,

Z entweder ein Wasserstoffatom und in diesem Fall T ebenfalls ein Wasserstoffatom oder Z

zusammen mit T eine Kette - $(CH_2)_n$-CH(E)- oder $(CH_2)_n$-CH($CH_2$)E'), worin n eine ganze Zahl mit einem Wert von 0, 1, 2 oder 3 mit der Maßgabe, daß in diesem Fall Z von einem Kohlenstoffatom getragen wird, das zu einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe darstellen,

wobei der im Zusammenhang mit den Ausdrücken Phenylgruppe, Heteroarylgruppe und Phenylalkylgruppe bei der Definition von $R_7$ und $R_8$ und E und E' benutzte Begriff "substituiert" bedeutet, daß diese Gruppen durch einen oder mehrere Substituenten ausgewählt aus Niedrigalkylgruppen, Niedrigalkoxygruppen, Halogenatomen, Trifluormethylgruppen oder Hydroxylgruppen substituiert sein können, und

$R_2$, $R_3$ und $R_4$, die gleichartig oderverschieden sein können, unabhängigvoneinander Hydroxylgruppen, Niedrigalkoxygruppen oder Niedrigalkylgruppen mit der Maßgabe bedeuten, daß

. wenn X ein Sauerstoffatom, Y eine Einfachbindung und Z ein Wasserstoffatom darstellen, mindestens eine der Gruppen $R_2$, $R_3$ und $R_4$ von einer Methoxygruppe verschieden ist.

. wenn X ein Sauerstoffatom, Y eine Gruppe $CR_7R_8$ und Z ein Wasserstoffatom darstellen, mindestens eine der Gruppen $R_2$, $R_3$ und $R_4$ keine Alkylgruppe darstellt,

deren Enantiomere, Epimere, Diastereoisomere sowie, wenn $R_1$ ein Wasserstoffatom darstellt oder wenn mindestens eine der Gruppen $R_2$, $R_3$ und $R_4$ eine Hydroxylgruppe darstellt, deren Additionssalze mit einer pharmazeutisch annehmbaren Base,

wobei es sich versteht, daß unter Niedrigalkylgruppen oder Niedrigalkoxygruppen geradkettige oder verzweigte Gruppen mit 1 bis 6 Kohlenstoffatomen und unter Heteroarylgruppen nicht-gesättigte mono- oder bicyclische Gruppen, die 5 bis 12 von Wasserstoff verschiedene Atome aufweisen und in einem Kohlenstoffskelett 1 bis 3 Heteroatome ausgewählt aus Stickstoff-, Sauerstoff- oder Schwefelatomen aufweisen, zu verstehen sind.

**2.** Verbindungen nach Anspruch 1, worin $R_3$ eine Hydroxylgruppe und $R_2$ und $R_4$ jeweils gleichzeitig eine tert.-Butylgruppe darstellen, deren Isomeren und deren Additionssalze mit einer pharmazeutisch annehmbaren Base.

**3.** Verbindungen nach Anspruch 1, worin Z zusammen mit T eine Kette $(CH_2)_n$-CH(E) oder $(CH_2)_n$-CH-($CH_2$E') bilden, worin n eine ganze Zahl mit einem Wert von 0, 1, 2 oder 3, mit der Maßgabe, daß in diesem Fall Z von einem Kohlenstoffatom getragen wird, das zu einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe darstellen, deren Enantiomere, Epimere, Diastereoisomere sowie, wenn $R_1$ ein Wasserstoffatom darstellt, oder wenn mindestens eine der Gruppen $R_2$, $R_3$ und $R_4$ eine Hydroxylgruppe darstellt, deren Additionssalze mit einer pharmazeutisch annehmbaren Base.

**4.** Verbindungen nach Anspruch 1, worin Z und T gleichzeitig Wasserstoffatme bedeuten, deren Isomere sowie, wenn $R_1$ ein Wasserstoffatom darstellt und mindestens eine der Gruppen $R_2$, $R_3$ und $R_4$ eine Hydroxylgruppe darstellt, deren Additionssalze mit einer pharmazeutisch annehmbaren Base.

**5.** Verbindungen nach Anspruch 1, worin Y eine Einfachbindung oder eine $CH_2$-Gruppe bedeutet, deren Isomere sowie, wenn $R_1$ ein Wasserstoffatom und mindestens eine der Gruppen $R_2$, $R_3$ und $R_4$ eine Hydroxylgruppe darstellen, deren Additionssalze mit einer pharmazeutisch annehmbaren Base.

**6.** Verbindungen nach Anspruch 1,worin X ein Sauerstoff- oder ein Schwefelatom darstellt, deren Isomere sowie, wenn $R_1$ ein Wasserstoffatom und mindestens eine der Gruppen $R_2$, $R_3$ und $R_4$ eine Hydroxylgruppe darstellen, deren Additionssalze mit einer pharmazeutisch annehmbaren Base.

**7.** Verbindungen nach Anspruch 3, worin E eine Phenylgruppe darstellt, die gegebenenfalls durch eine oder mehrere Gruppen ausgewählt aus Hydroxylgruppen und Niedrigalkylgruppen substituiert ist, deren Isomere und deren Additionssalze mit einer pharmazeutisch annehmbaren Base.

**8.** Verbindung nach Anspruch 1, nämlich 7-(3',5'-Di-tert.-butyl-4'-hydroxycinnamoyl)-[1,4]benzothiazin-3-on, dessen Isomere und dessen Additionssalze mit einer pharmazeutisch annehmbaren Base.

**9.** Verbindung nach Anspruch 1, nämlich 6-(3',5'-Di-tert.-butyl-4'-hydroxycinnamoyl)-benzoxazolinon, dessen Isomere und dessen Additionssalze mit einer pharmazeutisch annehmbaren Base.

**10.** Verbindung nach Anspruch 1, nämlich 2,3-Dihydro-5-phenyl-6-(3',5'-di-tert.-butyl-4'-hydroxy-benzyliden)-cyclopenta[f]benzoxazol-2,7-dion,dessen Isomere und dessen Additionssalze mit einer pharmazeutisch annehmbaren Base.

**11.** Verbindung nach Anspruch 1, nämlich [4H]-2,3-Dihydro-3,8-dioxo-2,2,4-trimethyl-6-(3',5'-di-tert.-butyl-4'-hydroxy-phenyl)-7(3',5'-di-tert.-butyl-4'-hydroxybenzyliden)-cyclopenta[g]-1,4-benzothiazin, dessen Isomere und dessen Additionssalze mit einer pharmazeutisch annehmbaren Base.

**12.** Verbindung nach Anspruch 1, nämlich [4H]-2,3-Dihydro-3,8-dioxo-4-methyl-6-phenyl-7-(3'5'-di-tert.-butyl-4'-hydroxy-benzyliden)-cyclopenta[g]-1,4-benzothiazin, dessen Isomere und dessen Additionssalze mit einer pharmazeutisch annehmbaren Base.

**13.** Verbindung nach Anspruch 1, nämlich 2,3-Dihydro-3-methyl-5-phenyl-6-(3',5'-di-tert.-butyl-4'-hydroxy-benzyliden)-cyclopenta[f]benzoxazol-2,7-dion, dessen Isomere und dessen Additionssalze mit einer pharmazeutisch annehmbaren Base.

**14.** Verbindung nach Anspruch 1, nämlich 2,3-Dihydro-5-phenyl-6-(3',5'-di-tert.-butyl-4'-hydroxy-benzyliden)-cyclopenta[f]benzothiazol-2,7-dion, dessen Isomere und dessen Additionssalze mit einer pharmazeutisch annehmbaren Base.

**15.** Verfahren zur Herstellung der Verbindungen der Formel (I) nach Anspruch 1, **dadurch gekennzeichnet**, daß man eine Verbindung der Formel (II):

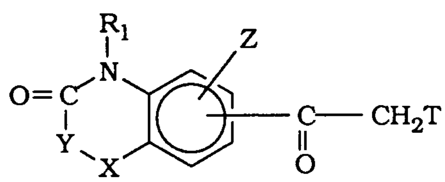

(II)

in der $R_1$, X, Y, Z und T die bezuglich der Formel (I) angegebenen Bedeutungen besitzen, in saurem organischem Medium mit einem Aldehyd der Formel (III):

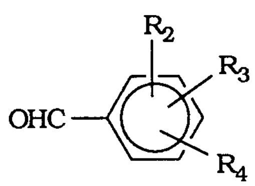

(III)

in der $R_2$, $R_3$ und $R_4$ die bezuglich der Formel (I) angegebenen Bedeutungen besitzen, umsetzt, so daß man nach der eventuellen Neutralisation des Reaktionsmediums ein Derivat der Formel (I) erhält, welches man erforderlichenfalls mit einer aus Chromatographie und Kristallisation ausgewählten Methode reinigt, gegebenenfalls in die Isomeren auftrennt und gewünschtenfalls mit einer pharmazeutisch annehmbaren Base in ein Salz überführt, wenn $R_1$ ein Wasserstoffatom oder mindestens eine der Gruppen $R_2$, $R_3$ und $R_4$ eine Hydroxylgruppe darstellen.

**16.** Verbindungen der Formel (II):

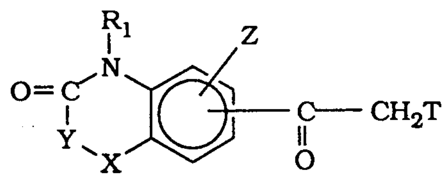

(II)

in der:

$R_1$ ein Wasserstoffatom oder eine Niedrigalkylgruppe,

X

. ein Sauerstoff- oder Schwefelatom,

. eine $CH_2$-Gruppe mit der Maßgabe, daß in diesem Fall Y ein Sauerstoff-oder Schwefelatom darstellt,

Y

. eine Einfachbindung oder eine Gruppe $CR_7R_8$, in der $R_7$ und $R_8$, die gleichartig oder verschieden sein können, unabhängig voneinander Wasserstoffatome, Niedrigalkylgruppen, Phenylgruppen, substituierte Phenylgruppen, Phenylalkylgruppen oder substituierte Phenylalkylgruppen darstellen,

. ein Sauerstoff- oder Schwefelatom mit der Maßgabe, daß in diesem Fall X eine $CH_2$-Gruppe darstellt, und

Z zusammen mit T eine Kette $-(CH_2)_n-CH(E)-$ oder $(CH_2)_n-CH(CH_2)E')$, worin n eine ganze Zahl mit einem Wert von 0, 1, 2 oder 3 mit der Maßgabe, daß in diesem Fall Z von einem Kohlenstoffatom getragen wird, das zu einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe darstellen, bedeuten, mit Ausnahme der Derivate, worin X ein Sauerstoffatom, Y eine Einfachbindung, E eine Methylgruppe oder E' ein Wasserstoffatom darstellen, sowie, wenn $R_1$ ein Wasserstoffatom darstellt oder wenn mindestens eine der Gruppen $R_2$, $R_3$ und $R_4$ eine Hydroxylgruppe darstellt, deren Additionssalze mit einer Base, die als Zwischenprodukte für die Synthese der Verbindungen der Formel (I) geeignet sind,

wobei der bezüglich der Begriffe Phenylgruppe. Heteroarylgruppe und Phenylalkylgruppe bei der Definition von $R_7$ und $R_8$ und E und E' benutzte Begriff "substituiert" bedeutet, daß diese Gruppen durch einen oder mehrere Substituenten ausgewählt aus Niedrigalkylgruppen, Niedrigalkoxygruppen, Halogenatomen, Trifluormethylgruppen oder Hydroxylgruppen substituiert sein können.

**17.** Pharmazeutische Zubereitung enthaltend als Wirkstoff mindestens eine Verbindung nach Anspruch 1 in Kombination mit einem oder mehreren inerten, nichttoxischen, pharmazeutisch annehmbaren Trägermaterialien oder Bindemitteln.

**18.** Pharmazeutische Zubereitung nach Anspruch 17, enthaltend mindestens einen Wirkstoff nach einem der Ansprüche 1 bis 15 für die Behandlung von Entzündungszuständen, der Atherosklerose, zur Vorbeugung und zur Behandlung von Plättchenstörungen bei Hypercholesterinämien, Hypertriglyceridämien und beim nicht insulinabhängigen Diabetes sowie seinen Komplikationen.

**Patentansprüche für folgenden Vertragsstaat : ES**

**1.** Verfahren zur Herstellung der Verbindungen der Formel (I):

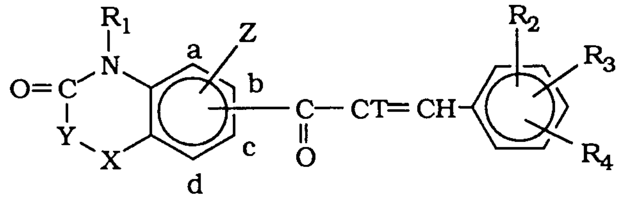

(I)

in der:

$R_1$ ein Wasserstoffatom oder eine Niedrigalkylgruppe,

X

. ein Sauerstoff- oder Schwefelatom,

. eine $CH_2$-Gruppe mit der Maßgabe, daß in diesem Fall Y ein Sauerstoff-oder Schwefelatom darstellt,

Y

. eine Einfachbindung oder eine Gruppe $CR_7R_8$, in der $R_7$ und $R_8$, die gleichartig oder verschieden sein können, unabhängig voneinander Wasserstoffatome, Niedrigalkylgruppen, Phenylgruppen, substituierte Phenylgruppen, Phenylalkylgruppen oder substituierte Phenylalkylgruppen darstellen,

. ein Sauerstoff- oder Schwefelatom mit der Maßgabe, daß in diesem Fall X eine $CH_2$-Gruppe darstellt,

Z entweder ein Wasserstoffatom und in diesem Fall T ebenfalls ein Wasserstoffatom oder Z zusammen mit T eine Kette $-(CH_2)_n-CH(E)-$ oder $(CH_2)_n-CH(CH_2)E')$, worin n eine ganze Zahl mit einem Wert von 0, 1, 2 oder 3 mit der Maßgabe, daß in diesem Fall Z von einem Kohlenstoffatom getragen wird, das zu einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe darstellen,

wobei der im Zusammenhang mit den Ausdrücken Phenylgruppe, Heteroarylgruppe und Phenylalkylgruppe bei der Definition von $R_7$ und $R_8$ und E und E' benutzte Begriff "substituiert" bedeutet, daß diese Gruppen durch einen oder mehrere Substituenten ausgewählt aus Niedrigalkylgruppen, Niedrigalkoxygruppen, Halogenatomen, Trifluormethylgruppen oder Hydroxylgruppen substituiert sein können, und

$R_2$, $R_3$ und $R_4$, die gleichartig oder verschieden sein können, unabhängig voneinander Hydroxylgruppen, Niedrigalkoxygruppen oder Niedrigalkylgruppen mit der Maßgabe bedeuten, daß

. wenn X ein Sauerstoffatom, Y eine Einfachbindung und Z ein Wasserstoffatom darstellen, mindestens eine der Gruppen $R_2$, $R_3$ und $R_4$ von einer Methoxygruppe verschieden ist,

. wenn X ein Sauerstoffatom, Y eine Gruppe $CR_7R_8$ und Z ein Wasserstoffatom darstellen, mindestens eine der Gruppen $R_2$, $R_3$ und $R_4$ keine Alkylgruppe darstellt,

deren Enantiomere, Epimere, Diastereoisomere sowie, wenn $R_1$ ein Wasserstoffatom darstellt oder wenn mindestens eine der Gruppen $R_2$, $R_3$ und $R_4$ eine Hydroxylgruppe darstellt, deren Additionssalze mit einer pharmazeutisch annehmbaren Base,

wobei es sich versteht, daß unter Niedrigalkylgruppen oder Niedrigalkoxygruppen geradkettige oder verzweigte Gruppen mit 1 bis 6 Kohlenstoffatomen und unter Heteroarylgruppen nicht-gesättigte mono- oder bicyclische Gruppen, die 5 bis 12 von Wasserstoff verschiedene Atome aufweisen und In einem Kohlenstoffskelett 1 bis 3 Heteroatome ausgewählt aus Stickstoff-, Sauerstoff- oder Schwefelatomen aufweisen, zu verstehen sind,

**dadurch gekennzeichnet**, daß man eine Verbindung der Formel (II):

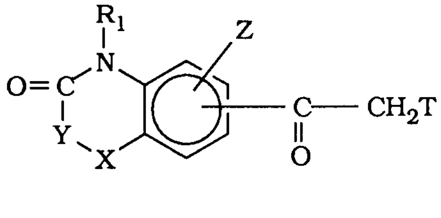

(II)

In der $R_1$, X, Y, Z und T die bezüglich der Formel (I) angegebenen Bedeutungen besitzen, in saurem organischem Medium mit einem Aldehyd der Formel (III):

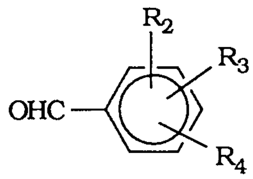

(III)

in der $R_2$, $R_3$ und $R_4$ die bezüglich der Formel (I) angegebenen Bedeutungen besitzen, umsetzt, so daß man nach der eventuellen Neutralisation des Reaktionsmediums ein Derivat der Formel (I) erhält, welches man erforderlichenfalls mit einer aus Chromatographie und Kristallisation ausgewählten Methode reinigt, gegebenenfalls in die Isomeren auftrennt und gewünschtenfalls mit einer pharmazeutisch annehmbaren Base in ein Salz überführt, wenn $R_1$ ein Wasserstoffatom oder mindestens eine der Gruppen $R_2$, $R_3$ und $R_4$ eine Hydroxylgruppe darstellen.

**2.** Verfahren zur Herstellung der Verbindungen der Formel (II) nach Anspruch 1 worin Z mit T eine Kette $-(CH_2)_n-CH(E)$ oder $(CH_2)_n-CH(CH_2E')$ bildet, mit Ausnahme der Derivate, worin X ein Sauerstoffatom, Y eine Einfachbindung, E eine Methylgruppe oder E' ein Wasserstoffatom bedeuten,
**dadurch gekennzeichnet**, daß man ein Derivat der Formel (IV):

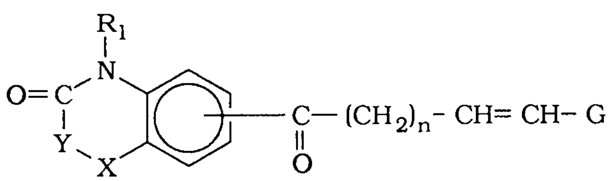

(IV)

in der n, Y, X und $R_1$ die bezüglich der Formel (I) angegebenen Bedeutungenbesitzen und G E oder $CH_2E'$ darstellt.
In saurem Medium cyclisiert,
welches Derivat der Formel (IV) man

- entweder, wenn Y eine Einfachbindung darstellt, durch Acylieren eines Derivats der Formel (V):

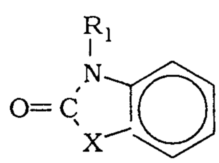

(V)

in der $R_1$ und X die bezüglich der Formel (I) angegebenen Bedeutungen besitzen, in saurem Medium mit einem Säurechlorid der Formel (VI):

$ClOC - (CH_2)_nCH = CH - E$ (VI)

in der n und E die bezüglich der Formel (I) angegebenen Bedeutungen besitzen, oder mit einem Derivat der Formel (VII):

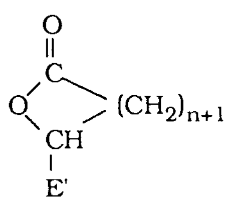

(VII)

in der n und E' die bezüglich der Formel (I) angegebenen Bedeutungen besitzen,

- oder, wenn n = 0 bedeutet , durch Kondensation eines Derivats der Formel (VIII):

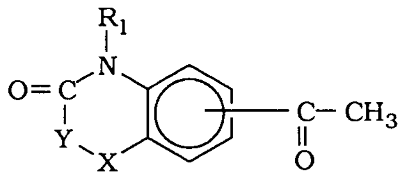

(VIII)

in der X, Y, $R_1$ und n die bezüglich der Formel (I) angegebenen Bedeutungen besitzen, mit einem Aldehyd der Formel (IX):

G - CHO (IX)

in der G die oben angegebenen Bedeutungen besitzt,

- oder, wenn G ein Wasserstoffatom und n = 0 bedeuten, durch Dehydrohalogenierung eines Derivats der Formel (X):

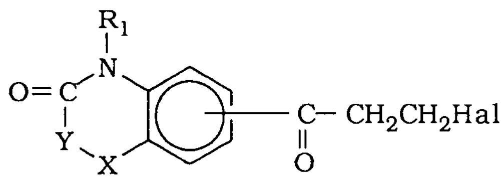

(X)

in der $R_1$, X, Y und n die oben angegebenen Bedeutungen besitzen und Hal ein Halogenatom darstellt, in alkalischem Medium

erhält, wobei man die Verbindungen gegebenenfalls reinigt und/oder in die Isomeren auftrennt.

**3.** Verfahren nach Anspruch 1 zur Herstellung von Verbindungen der Formel (I), in der $R_3$ eine Hydroxylgruppe und $R_2$ und $R_4$ jeweils gleichzeitig eine tert.-Butylgruppe bedeuten, von deren Isomeren und von deren Additionssalzen mit einer pharmazeutisch annehmbaren Base.

**4.** Verfahren nach Anspruch 1 zur Herstellung von Verbindungen der Formel (I), in der Z mit T eine Kette $(CH_2)_n$-CH(E) oder $(CH_2)_n$-CH($CH_2$E') bildet, worin n eine ganze Zahl mit einem Wert von 0, 1, 2 oder 3, mit der Maßgabe, daß in diesem Fall Z von einem Kohlenstoffatom getragen wird, das einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe bedeuten,
von deren Enantiomeren, Epimeren und Diastereoisomeren sowie, wenn $R_1$ ein Wasserstoffatom oder wenn mindestens eine der Gruppen $R_2$, $R_3$ und $R_4$ eine Hydroxylgruppe darstellen, von deren Additionssalzen mit einer pharmazeutisch annehmbaren Base.

**5.** Verfahren nach Anspruch 1 zur Herstellung der Verbindungen der Formel (I), in der Z und T gleichzeitig ein Wasserstoffatom bedeuten, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer pharmazeutisch annehmbaren Base.

**6.** Verfahren nach Anspruch 1 zur Herstellung von Verbindungen der Formel (I), in der Y eine Einfachbindung oder eine $CH_2$-Gruppe darstellt, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer pharmazeutisch annehmbaren Base.

**7.** Verfahren nach Anspruch 1 zur Herstellung der Verbindungen der Formel (I) in der X ein Sauerstoff- oder Schwefelatom darstellt, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer pharmazeutisch annehmbaren Base.

**8.** Verfahren nach Anspruch 4 zur Herstellung von Verbindungen der Formel (I), in der E eine Phenylgruppe darstellt, die gegebenenfalls durch eine oder mehrere Gruppen ausgewählt aus Hydroxylgruppen und Niedrigalkylgruppen substituiert ist, von deren Isomeren und von deren Additionssalzen mit einer pharmazeutisch annehmbaren Base.

**9.** Verfahren nach Anspruch 1 zur Herstellung der Verbindung der Formel (I), nämlich 7-(3',5'-Di-tert.-butyl-4'-hydroxy-cinnamoyl)-[1,4]benzothiazin-3-on, von dessen Isomeren und von dessen Additionssalzen mit einer pharmazeutisch annehmbaren Base.

**10.** Verfahren nach Anspruch 1 zur Herstellung der Verbindung der Formel (I), nämlich 6-(3',5'-Di-tert.-butyl-4'-hydroxy-cinnamoyl)-benzoxazolinon, von dessen Isomeren und von dessen Additionssalzen mit einer pharmazeutisch annehmbaren Base.

**11.** Verfahren nach Anspruch 1 zur Herstellung der Verbindung der Formel (I), nämlich 2,3-Dihydro-5-phenyl-6-(3',5'-di-tert.-butyl-4'-hydroxy-benzyliden)-cyclopenta[f]benzoxazol-2,7-dion, von dessen Isomeren und von dessen Additionssalzen mit einer pharmazeutisch annehmbaren Base.

**12.** Verfahren nach Anspruch 1 zur Herstellung der Verbindung der Formel (I), nämlich [4H]-2,3-Dihydro-3,8-dioxo-2,2,4-trimethyl-6-(3',5'-di-tert.-butyl-4'-hydroxy-phenyl)-7-(3',5'-di-tert.-butyl-4'-hydroxybenzyliden)-cyclopenta[g]-1,4-benzothiazin, von dessen Isomeren und von dessen Additionssalzen mit einer pharmazeutisch annehmbaren Base.

**13.** Verfahren nach Anspruch 1 zur Herstellung der Verbindung der Formel (I), nämlich [4H]-2,3-Dihydro-3,8-dioxo-4-methyl-6-phenyl-7-(3',5'-di-tert.-butyl-4'-hydroxy-benzyliden)-cyclopenta[g]-1,4-benzothiazinvon dessen Isomeren und von dessen Additionssalzen mit einer pharmazeutisch annehmbaren Base.

**14.** Verfahren nach Anspruch 1 zur Herstellung der Verbindung der Formel (I), nämlich 2,3-Dihydro-3-methyl-5-phenyl-6-(3',5'-di-tert.-butyl-4'-hydroxy-benzyliden)-cyclopenta[f]benzoxazol-2,7-dion, von dessen Isomeren und von dessen Additionssalzen mit einer pharmazeutisch annehmbaren Base.

**15.** Verfahren nach Anspruch 1 zur Herstellung der Verbindung der Formel (I), nämlich 2,3-Dihydro-5-phenyl-6-(3',5'-di-tert.-butyl-4'-hydroxy-benzyliden)-cyclopenta[f]benzothiazol-2,7-dion, von dessen Isomeren und von dessen Additionssalzen mit einer pharmazeutisch annehmbaren Base.

**16.** Verfahren zur Herstellung einer pharmazeutischen Zubereitung, die als Wirkstoffmindestens eine Verbindung erhalten nach Anspruch 1 in Kombination mit einem oder mehreren inerten, nichttoxischen, pharmazeutisch annehmbaren Trägermaterialien oder Bindemitteln enthält.

**17.** Verfahren zur Herstellung der pharmazeutischen Zubereitung nach Anspruch 16, enthaltend mindestens einen Wirkstoff erhalten nach einem der Ansprüche 1 bis 15, für die Behandlung von Entzündungen, zur Vorbeugung und die Behandlung von Plättchenstörungen bei Hypercholesterinämien, Hypertriglyceridämien, dem nicht insulinabhängigen Diabetes sowie seinen Komplikationen.

**Patentansprüche für folgenden Vertragsstaat : GR**

**1.** Verfahren zur Herstellung der Verbindungen der Formel (I):

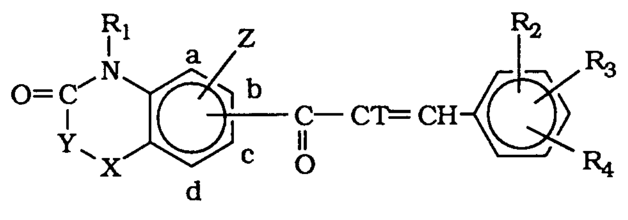

(I)

in der:

$R_1$ ein Wasserstoffatom oder eine Niedrigalkylgruppe.

X
. ein Sauerstoff- oder Schwefelatom,
. eine $CH_2$-Gruppe mit der Maßgabe, daß in diesem Fall Y ein Sauerstoff-oder Schwefelatom darstellt.

Y
. eine Einfachbindung oder eine Gruppe $CR_7R_8$, in der $R_7$ und $R_8$, die gleichartig oder verschieden sein können, unabhängig voneinander Wasserstoffatome, Niedrigalkylgruppen, Phenylgruppen, substituierte Phenylgruppen, Phenylalkylgruppen oder substituierte Phenylalkylgruppen darstellen,
. ein Sauerstoff- oder Schwefelatom mit der Maßgabe, daß in diesem Fall X eine $CH_2$-Gruppe darstellt,

Z entweder ein Wasserstoffatom und in diesem Fall T ebenfalls ein Wasserstoffatom oder Z zusammen mit T eine Kette $-(CH_2)_n-CH(E)-$ oder $(CH_2)_n-CH(CH_2)E')$, worin n eine ganze Zahl mit einem Wert von 0, 1, 2 oder 3 mit der Maßgabe, daß in diesem Fall Z von einem Kohlenstoffatom getragen wird, das zu einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe darstellen,

wobei der im Zusammenhang mit den Ausdrücken Phenylgruppe, Heteroarylgruppe und Phenylalkylgruppe bei der Definition von $R_7$ und $R_8$ und E und E' benutzte Begriff "substituiert" bedeutet, daß diese Gruppen durch einen oder mehrere Substituenten ausgewählt aus Niedrigalkylgruppen, Niedrigalkoxygruppen, Halogenatomen, Trifluormethylgruppen oder Hydroxylgruppen substituiert sein können, und

$R_2$, $R_3$ und $R_4$, die gleichartig oder verschieden sein können, unabhängig voneinander Hydroxylgruppen, Niedrigalkoxygruppen oder Niedrigalkylgruppen mit der Maßgabe bedeuten, daß
. wenn X ein Sauerstoffatom, Y eine Einfachbindung und Z ein Wasserstoffatom darstellen, mindestens eine der Gruppen $R_2$, $R_3$ und $R_4$ von einer Methoxygruppe verschieden ist,
. wenn X ein Sauerstoffatom, Y eine Gruppe $CR_7R_8$ und Z ein Wasserstoffatom darstellen, mindestens eine der Gruppen $R_2$, $R_3$ und $R_4$ keine Alkylgruppe darstellt,

deren Enantiomere, Epimere, Diastereoisomere sowie, wenn $R_1$ ein Wasserstoffatom darstellt oder wenn mindestens eine der Gruppen $R_2$, $R_3$ und $R_4$ eine Hydroxylgruppe darstellt, deren Additionssalze mit einer pharmazeutisch annehmbaren Base,
wobei es sich versteht, daß unter Niedrigalkylgruppen oder Niedrigalkoxygruppen geradkettige oder verzweigte Gruppen mit 1 bis 6 Kohlenstoffatomen und unter Heteroarylgruppen nicht-gesättigte mono- oder bicyclische Gruppen, die 5 bis 12 von Wasserstoff verschiedene Atome aufweisen und in einem Kohlenstoffskelett 1 bis 3 Heteroatome ausgewählt aus Stickstoff-, Sauerstoff- oder Schwefelatomen aufweisen, zu verstehen sind,
**dadurch gekennzeichnet**, daß man eine Verbindung der Formel (II):

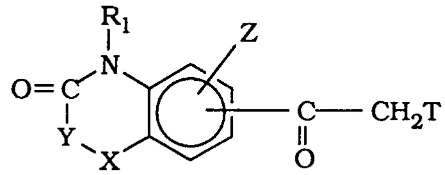 (II)

in der $R_1$, X, Y, Z und T die bezüglich der Formel (I) angegebenen Bedeutungen besitzen,
in saurem organischem Medium mit einem Aldehyd der Formel (III):

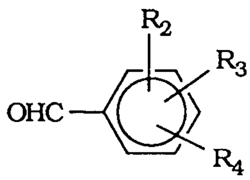

(III)

in der $R_2$, $R_3$ und $R_4$ die bezüglich der Formel (I) angegebenen Bedeutungen besitzen, umsetzt, so daß man nach der eventuellen Neutralisation des Reaktionsmediums ein Derivat der Formel (I) erhält, welches man erforderlichenfalls mit einer aus Chromatographie und Kristallisation ausgewählten Methode reinigt, gegebenenfalls in die Isomeren auftrennt und gewünschtenfalls mit einer pharmazeutisch annehmbaren Base in ein Salz überführt, wenn $R_1$ ein Wasserstoffatom oder mindestens eine der Gruppen $R_2$, $R_3$ und $R_4$ eine Hydroxylgruppe darstellen.

**2.** Verfahren zur Herstellung der Verbindungen der Formel (II) nach Anspruch 1, worin Z mit T eine Kette $-(CH_2)_n-CH(E)$ oder $(CH_2)_n-CH(CH_2E')$ bildet, mit Ausnahme der Derivate, worin X ein Sauerstoffatom, Y eine Einfachbindung, E eine Methylgruppe oder E' ein Wasserstoffatom bedeuten,
**dadurch gekennzeichnet**, daß man ein Derivat der Formel (IV):

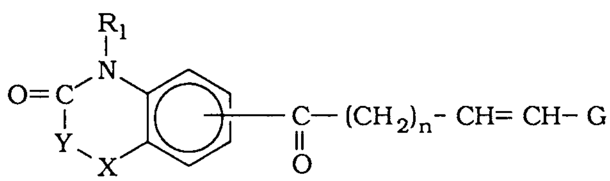

(IV)

in der n, Y, X und $R_1$ die bezüglich der Formel (I) angegebenen Bedeutungen besitzen und G E oder $CH_2E'$ darstellt,
in saurem Medium cyclisiert,
welches Derivat der Formel (IV) man
- entweder, wenn Y eine Einfachbindung darstellt, durch Acylieren eines Derivats der Formel (V):

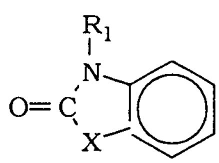

(V)

in der $R_1$ und X die bezüglich der Formel (I) angegebenen Bedeutungen besitzen, in saurem Medium mit einem Säurechlorid der Formel (VI):

$ClOC - (CH_2)_nCH = CH - E$ (VI)

in der n und E die bezüglich der Formel (I) angegebenen Bedeutungen besitzen, oder mit einem Derivat der Formel (VII):

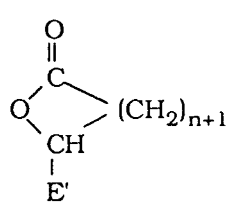

(VII)

in der n und E' die bezüglich der Formel (I) angegebenen Bedeutungen besitzen,

- oder, wenn n = 0 bedeutet, durch Kondensation eines Derivats der Formel (VIII):

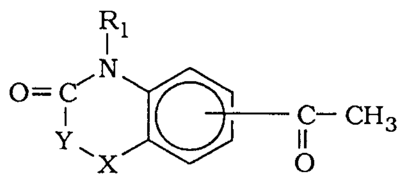

(VIII)

in der X, Y, $R_1$ und n die bezüglich der Formel (I) angegebenen Bedeutungen besitzen, mit einem Aldehyd der Formel (IX):

G - CHO (IX)

in der G die oben angegebenen Bedeutungen besitzt,
- oder, wenn G einWasserstoffatom und n = 0 bedeuten, durch Dehydrohalogenierung eines Derivats der Formel (X):

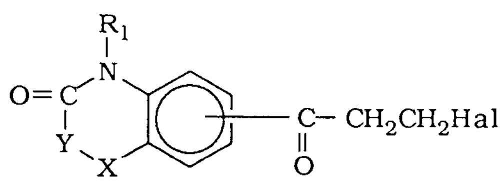

(X)

in der $R_1$, X, Y und n die oben angegebenen Bedeutungen besitzen und Hal ein Halogenatom darstellt, in alkalischem Medium

erhält, wobei man die Verbindungen gegebenenfalls reinigt und/oder in die Isomeren auftrennt.

3. Verfahren nach Anspruch 1 zur Herstellung von Verbindungen der Formel (I), in der $R_3$ eine Hydroxylgruppe und $R_2$ und $R_4$ jeweils gleichzeitig eine tert.-Butylgruppe bedeuten, von deren Isomeren und von deren Additionssalzen mit einer pharmazeutisch annehmbaren Base.

4. Verfahren nach Anspruch 1 zur Herstellung von Verbindungen der Formel (I), in der Z mit T eine Kette $(CH_2)_n$-CH(E) oder $(CH_2)_n$-CH($CH_2$E') bildet, worin n eine ganze Zahl mit einem Wert von 0, 1, 2 oder 3, mit der Maßgabe, daß in diesem Fall Z von einem Kohlenstoffatom getragen wird, das einem eine Acylgruppe tragenden Kohlenstoffatom benachbart ist, und E oder E' ein Wasserstoffatom, eine Niedrigalkylgruppe, eine Phenylgruppe, eine Heteroarylgruppe, eine substituierte Phenylgruppe oder eine substituierte Heteroarylgruppe bedeuten,
von deren Enantiomeren, Epimeren und Diastereoisomeren sowie, wenn $R_1$ ein Wasserstoffatom oder wenn mindestens eine der Gruppen $R_2$, $R_3$ und $R_4$ eine Hydroxylgruppe darstellen, von deren Additionssalzen mit einer pharmazeutisch annehmbaren Base.

5. Verfahren nach Anspruch 1 zur Herstellung der Verbindungen der Formel (I), in der Z und T gleichzeitig ein Wasserstoffatom bedeuten, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer pharmazeutisch annehmbaren Base.

6. Verfahren nach Anspruch 1 zur Herstellung von Verbindungen der Formel (I), in der Y eine Einfachbindung oder eine $CH_2$-Gruppe darstellt, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer pharmazeutisch annehmbaren Base.

7. Verfahren nach Anspruch 1 zur Herstellung der Verbindungen der Formel (I), in der X ein Sauerstoff- oder Schwefelatom darstellt, von deren Isomeren sowie, wenn $R_1$ ein Wasserstoffatom und mindestens eine der Gruppen $R_2$, $R_3$ und $R_4$ eine Hydroxylgruppe darstellen, von deren Additionssalzen mit einer

pharmazeutisch annehmbaren Base.

**8.** Verfahren nach Anspruch 4 zur Herstellung von Verbindungen der Formel (I), in der E eine Phenylgruppe darstellt, die gegebenenfalls durch eine oder mehrere Gruppen ausgewählt aus Hydroxylgruppen und Niedrigalkylgruppen substituiert ist, von deren Isomeren und von deren Additionssalzen mit einer pharmazeutisch annehmbaren Base.

**9.** Verfahren nach Anspruch 1 zur Herstellung der Verbindung der Formel (I), nämlich 7-(3',5'-Di-tert.-butyl-4'-hydroxy-cinnamoyl)-[1,4]benzothiazin-3-on, von dessen Isomeren und von dessen Additionssalzen mit einer pharmazeutisch annehmbaren Base.

**10.** Verfahren nach Anspruch 1 zur Herstellung der Verbindung der Formel (I), nämlich 6-(3',5'-Di-tert.-butyl-4'-hydroxy-cinnamoyl)-benzoxazolinon, von dessen Isomeren und von dessen Additionssalzen mit einer pharmazeutisch annehmbaren Base.

**11.** Verfahren nach Anspruch 1 zur Herstellung der Verbindung der Formel (I), nämlich 2,3-Dihydro-5-phenyl-6-(3',5'-di-tert.-butyl-4'-hydroxy-benzyliden)-cyclopenta[f]benzoxazol-2,7-dion, von dessen Isomeren und von dessen Additionssalzen mit einer pharmazeutisch annehmbaren Base.

**12.** Verfahren nach Anspruch 1 zur Herstellung der Verbindung der Formel (I), nämlich [4H]-2,3-Dihydro-3,8-dioxo-2,2,4-trimethyl-6-(3',5'-di-tert.-butyl-4'-hydroxy-phenyl)-7-(3',5'-di-tert.-butyl-4'-hydroxybenzyliden)-cyclopenta[g]-1,4-benzothiazin, von dessen Isomeren und von dessen Additionssalzen mit einer pharmazeutisch annehmbaren Base.

**13.** Verfahren nach Anspruch 1 zur Herstellung der Verbindung der Formel (I), nämlich [4H]-2,3-Dihydro-3,8-dioxo-4-methyl-6-phenyl-7-(3',5'-di-tert.-butyl-4'-hydroxy-benzyliden)-cyclopenta[g]-1,4-benzothiazin, von dessen Isomeren und von dessen Additionssalzen mit einer pharmazeutisch annehmbaren Base.

**14.** Verfahren nach Anspruch 1 zur Herstellung der Verbindung der Formel (I), nämlich 2,3-Dihydro-3-methyl-5-phenyl-6-(3',5'-di-tert.-butyl-4'-hydroxy-benzyliden)-cyclopenta[f]benzoxazol-2,7-dion, von dessen Isomeren und von dessen Additionssalzen mit einer pharmazeutisch annehmbaren Base.

**15.** Verfahren nach Anspruch 1 zur Herstellung der Verbindung der Formel (I), nämlich 2,3-Dihydro-5-phenyl-6-(3',5'-di-tert.-butyl-4'-hydroxy-benzyliden)-cyclopenta[f]benzothiazol-2,7-dion, von dessen Isomeren und von dessen Additionssalzen mit einer pharmazeutisch annehmbaren Base.

**16.** Verfahren zur Herstellung einer pharmazeutischen Zubereitung, die als Wirkstoffmindestens eine Verbindung erhalten nach Anspruch 1 in Kombination mit einem oder mehreren inerten, nichttoxischen, pharmazeutisch annehmbaren Trägermaterialien oder Bindemitteln enthält.

**17.** Verfahren zur Herstellung der pharmazeutischen Zubereitung nach Anspruch 16, enthaltend mindestens einen Wirkstoff erhalten nach einem der Ansprüche 1 bis 15, für die Behandlung von Entzündungen, zur Vorbeugung und die Behandlung von Plättchenstörungen bei Hypercholesterinämien, Hypertriglyceridämien, dem nicht insulinabhängigen Diabetes sowie seinen Komplikationen.